# FASCICULE DE BREVET EUROPEEN

(11) **EP 1 232 165 B1**
(45) Date de publication et mention de la délivrance du brevet: **07.05.2003**
(21) Numéro de dépôt: 00985306.0
(22) Date de dépôt: 23.11.2000
(51) Int. Cl.: C07F 9/6581, C12N 15/87, C07F 9/24

(54) **DENDRIMERES PHOSPHORES COMME AGENTS DE TRANSFECTION**
PHOSPHOR-ENTHALTENDE DENDRIMERE ALS TRANSFEKTIONSAGENTE
PHOSPHORUS-CONTAINING DENDRIMERS AS TRANSFECTION AGENTS

(30) Priorité: 25.11.1999 FR 9914864
(43) Date de publication de la demande: 21.08.2002
(73) Titulaire: CENTRE NATIONAL DE LA RECHERCHE SCIENTIFIQUE (CNRS), 75794 Paris Cedex 16 (FR)
(72) Inventeur: MAJORAL, Jean-Pierre, F-31520 Ramonville (FR); MEUNIER, Bernard, F-31320 Castanet (FR); CAMINADE, Anne-Marie, F-31400 Toulouse (FR); LOUP, Christophe, F-31000 Toulouse (FR); ZANTA-BOUSSIF, Maria-Antonietta, F-68300 Saint Louis (FR)
(74) Mandataire: Orès, Bernard
(86) Numéro de dépôt international: FR0003261
(87) Numéro de publication internationale: WO01038335

(56) Documents cités:
- WO-A-95/02397
- GALLIOT C.: "Regioselective stepwise growth of dendrimer units in the internal voids of a main dendrimer" SCIENCE., vol. 277, 26 septembre 1997 (1997-09-26), pages 1981-1984, XP000929654 AAAS. LANCASTER, PA., US cité dans la demande
- LARR C.: "Phosphorus-containing dendrimers: chemoselective functionalization of internal layers" JOURNAL OF THE AMERICAN CHEMICAL SOCIETY., vol. 120, no. 16, 29 avril 1998 (1998-04-29), pages 4029-4030, XP002144558 AMERICAN CHEMICAL SOCIETY, WASHINGTON, DC., US ISSN: 0002-7863 cité dans la demande
- PR VOT D.: "Phosphate-, phosphite-, ylide-, and phosphonate-terminated dendrimers" JOURNAL OF ORGANIC CHEMISTRY., vol. 62, no. 14, 11 juillet 1997 (1997-07-11), pages 4834-4841, XP002144559 AMERICAN CHEMICAL SOCIETY. EASTON., US ISSN: 0022-3263
- LOUP C.: "Preparation of water-soluble cationic phosphorus-containing dendrimers as DNA transfecting agents" CHEMISTRY - A EUROPEAN JOURNAL., vol. 5, no. 12, décembre 1999 (1999-12), pages 3644-3650, XP002144560 VCH PUBLISHERS., US ISSN: 0947-6539
- MARAVAL V.: "Rapid synthesis of phosphorus-containing dendrimers with controlled molecular architectures: first example of surface-block, layer-block, and segment-block dendrimers issued from the same dendron" JOURNAL OF THE AMERICAN CHEMICAL SOCIETY., vol. 122, no. 11, 22 mars 2000 (2000-03-22), pages 2499-2511, XP002144561 AMERICAN CHEMICAL SOCIETY, WASHINGTON, DC., US ISSN: 0002-7863

## Description

La présente invention est relative à des dendrimères phosphorés et à leurs applications, comme agents de transfection de gènes, *in vitro* et *in vivo*, y compris dans le traitement des maladies humaines et animales. Lesdits agents ou vecteurs sont notamment aptes à dispenser à des cellules cibles convenables, des séquences d'acide nucléique d'intérêt.

La thérapie génique est fondée sur l'administration thérapeutique d'acides nucléiques ; elle nécessite l'utilisation de vecteurs efficaces et sûrs pour le transfert des gènes thérapeutiques et son succès dépend donc de l'efficacité du transfert des gènes dans les cellules souhaitées.

De nombreuses compositions aptes à transfecter des cellules eucaryotes avec un matériel génétique sélectionné ont déjà été décrites et appartiennent essentiellement à deux grands types de vecteurs de transfection :
- les vecteurs de transfection viraux, qui sont efficaces mais qui présentent des limites d'utilisation : non-spécificité tissulaire, nécessité d'obtenir des constructions pour chaque gène d'intérêt, risques potentiels pour l'environnement qui conduisent à la mise en place d'infrastructures cliniques coûteuses et contraignantes pour le malade et le personnel, problèmes de réponse immunitaire, de production par recombinaison homologue de particules virales et d'effets oncogènes potentiels ;
- des agents non-viraux (vecteurs synthétiques), capables de promouvoir le transfert et l'expression de substances chimiques telles que l'ADN dans les cellules eucaryotes.

Ces vecteurs synthétiques doivent avoir essentiellement deux fonctions : condenser l'ADN à transfecter et promouvoir sa fixation cellulaire ainsi que son passage à travers la membrane plasmique et éventuellement les membranes nucléaires ; pour être efficaces, de tels vecteurs doivent donc mimer le fonctionnement des virus. Toutefois, il apparaît que les différents vecteurs proposés dans l'art antérieur ne présentent pas ces deux fonctions de manière optimale et peuvent en outre, selon les cas, être toxiques pour les cellules.

Parmi ces vecteurs non-viraux, on peut notamment citer les préparations de lipides cationiques, qui sont particulièrement cytotoxiques (15-18), les techniques d'encapsulation de l'ADN dans des liposomes (9-11) et les polymères polycationiques, tels que la poly(L-lysine), la protamine, le polyéthylèneimine (12) ou encore les dendrimères cationiques (polyamidoamines) (13, 14, 27), qui s'associent à l'ADN par des interactions électrostatiques multiples engendrant un processus de coopérativité, ce qui produit des particules appelées polyplexes, chacune donnant des efficacités de transfection variables suivant leur structure et le type cellulaire (25).

De manière plus précise, les dendrimères de type polyamidoamines (PAMAM) sont des polymères de structure sphérique et branchée ; ils sont solubles en solution aqueuse et présentent à leur surface une couche d'amines primaires ; ils sont isomoléculaires et fortement chargés à leur surface. Les différents dendrimères décrits présentent différents types de noyau central (*core*) : ammonium ou éthylènediamine (EDA), à partir desquels le processus de polymérisation est initié. Ces dendrimères PAMAM contiennent un nombre défini de groupes aminés à la surface du polymère qui sont chargés positivement à pH physiologique. Il a été montré que de telles molécules interagissent avec des anions tels que les acides nucléiques et que les complexes ADN/dendrimères sont capables de transfecter des cellules d'une manière similaire à celle observée avec les complexes ADN/polylysine, mais avec une efficacité meilleure, liée à leur solubilité et à leur structure. En particulier des dendrimères PAMAM G₃-G₅, comprenant comme noyau central, soit des groupes NH₃, soit des groupes EDA, sont capables de former des complexes stables avec de l'ADN dans des conditions physiologiques, et les dendrimères G₅-G₁₀ sont capables de transfecter des gènes. Le fait que la capacité de transfection soit limitée à ce groupe de dendrimères est sans doute liée au nombre de groupes aminés présents à leur surface et à leur forme sphérique. L'efficacité de ces derniers dépend des modifications chimiques de la structure dendrimérique initiale, par un traitement thermique qui conduit à une population de composés hétérodispersés (15, 21).

Depuis environ dix ans, la recherche sur la chimie des dendrimères s'est considérablement développée, du fait de leur structure (polymères polyfonctionnels ordonnés) et de leurs propriétés particulières liées à la présence d'un nombre important de fonctionnalités à leurs extrémités, qui fournissent un grand nombre de surfaces et d'interfaces et à la présence de zones vides, qui permettent l'encapsulation de molécules diverses ; cette recherche a conduit à la synthèse d'autres structures correspondant à des dendrimères contenant du phosphore, telles que celles décrites par C. Galliot et al. (18), par C. Larré et al. (19) ou par D. Prévôté et al. (28).

Toutefois, il ressort de la littérature que ces dendrimères phosphorés, non solubles dans l'eau, ne peuvent être utilisés comme agents de transfection.

Dans le cadre de leur recherche, les Inventeurs ont maintenant montré que de nouveaux dendrimères contenant du phosphore, qui sont fonctionnalisés au niveau des couches internes et qui comprennent des amines tertiaires protonées comme terminaisons présentent des propriétés particulièrement intéressantes, en tant que vecteurs de transfert d'acides nucléiques.

La présente invention a pour objet des dendrimères phosphorés polycationiques, caractérisés en ce qu'ils sont constitués :
- d'une couche centrale sous la forme d'un noyau P₀ comprenant 2 à 8 groupements fonctionnalisés et notamment le groupement de formule générale Ia (aussi dénommé N₃P₃) : 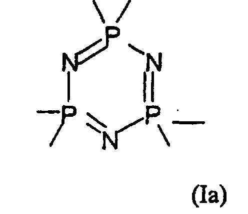 ou le groupement de formule générale Ib : 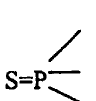
- de n couches intermédiaires, identiques ou différentes, chacune desdites couches intermédiaires étant constituée d'unités P1 répondant à la formule II : 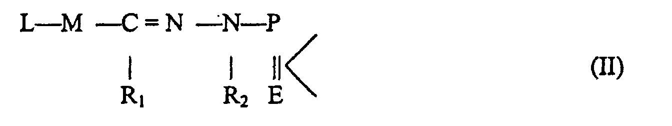
dans laquelle :
**L** est un atome d'oxygène, de phosphore ou de soufre,
**M** représente l'un des groupes suivants :
   - un groupe aromatique di-, tri- ou tétrasubstitué par des groupes alkyles, des groupes alcoxy, des groupements insaturés du type oléfinique en C₁-C₁₂, azoïque, acétylènique, tous ces groupes pouvant incorporer ou non des atomes de phosphore, d'oxygène, d'azote, de soufre ou des halogènes, ou
   - un groupe alkyle ou alcoxy comportant plusieurs substituant tels que définis lorsque M est un groupe aromatique,
**R**_{**1**} **et R**_{**2**}**,** qui peuvent être identiques ou différents, représentent un atome d'hydrogène ou l'un des groupes suivants : alkyle, alcoxy, aryle, comportant ou non des atomes de phosphore, d'oxygène, de soufre, d'azote ou des halogènes avec R₂ étant le plus souvent différent de R₁,
**n** est un nombre entier compris entre 1 et 11,
**E** est un atome d'oxygène, de soufre ou d'azote, ledit atome d'azote pouvant être lié à un groupe alkyle, alcoxy ou aryle, tous ces groupes pouvant incorporer ou non des atomes de phosphore, d'oxygène, d'azote, de soufre ou des halogènes,
   - une couche externe constituée d'unités P2, identiques ou différentes, et répondant à la formule III : 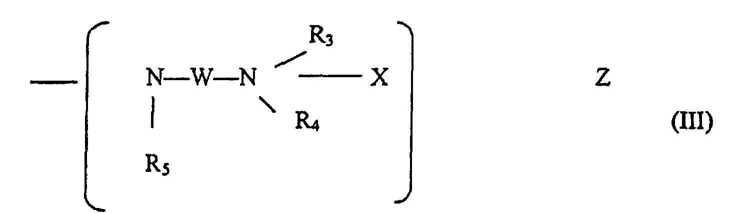
   dans laquelle :
**R**_{**5**} représente un atome d'hydrogène ou l'un des groupes suivants : alkyle, alcoxy, aryle, ces groupes comportant ou non des atomes de phosphore, d'oxygène, d'azote, de soufre ou des halogènes,
**W** représente l'un des groupes suivants : alkyle, alcoxy, aryle, tous ces groupes comportant où non des atomes de phosphore, d'oxygène, d'azote, de soufre ou des halogènes,
**R**_{**3**} et **R**_{**4**}, qui peuvent être identiques ou différents, représentent un groupe alkyle en C₁-C₅,
**X** représente un atome d'hydrogène ou un groupe alkyle en C₁-C₅ ou est absent, et
**Z** représente un ion halogénure, un groupe alkylCOO⁻ ou tout autre groupement anionique comportant des atomes de carbone, d'oxygène, de soufre, d'azote, de phosphore ou d'halogènes, ou est absent.

Un groupe de formule II préféré est notamment le groupe suivant :

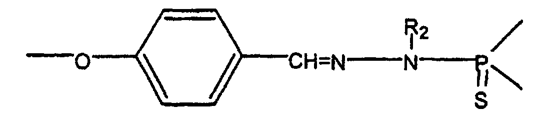

Un groupe de formule III préféré est notamment le groupe suivant : 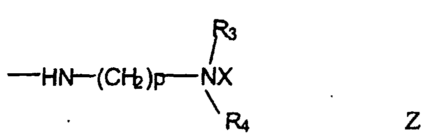 où p = 1 à 5

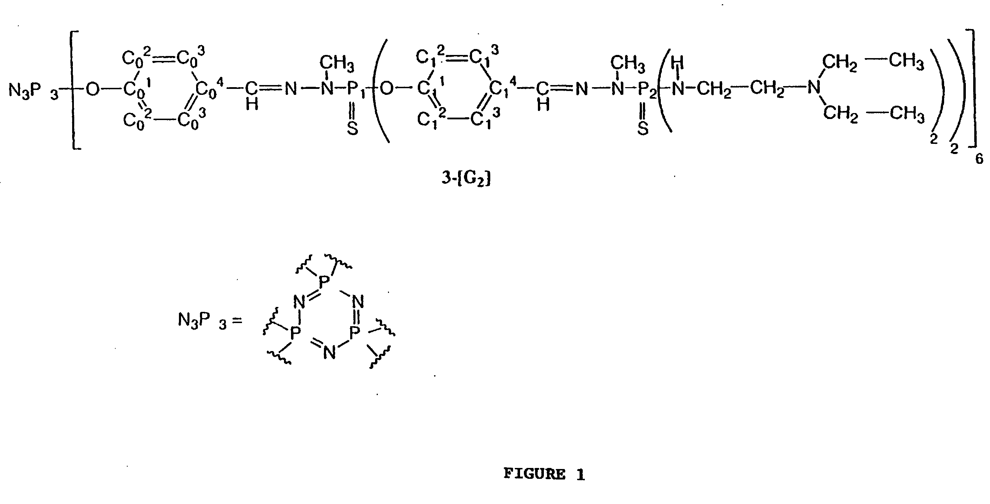

De tels composés représentent des dendrimères et sont notamment représentés aux figures 1 à 3.

L'expression « alcoxy » désigne les radicaux de formule générale R'O-, par exemple les groupements méthoxy, éthoxy, propoxy, isopropoxy, butoxy, isobutoxy, tertiobutoxy.

L'expression « alkyle » désigne les radicaux à chaîne linéaire ou ramifiée contenant jusqu'à 8 atomes de carbone. Parmi ces radicaux, on peut citer les radicaux méthyle, éthyle, propyle, isopropyle, butyle, isobutyle, sec-butyle, tert-butyle, pentyle, hexyle, heptyle, octyle.

L'expression «aryle» désigne, par exemple, un radical phényle éventuellement substitué par un ou plusieurs radicaux alcoyle, alcoxyle ou par un atome de chlore, de brome ou de fluor, ou un radical aromatique hétérocyclique à 5 ou 6 chaînons contenant 1 à 2 hétéroatomes tels que l'azote ou l'oxygène. Parmi ces radicaux aryles, on peut citer les radicaux phényle (o-, m-, ou p-), méthoxyphényle (3,4-, 2,6-, 2,3-), diméthoxyphényle (o-, m-, ou p-), tolyle, thiényle, pyridyle.

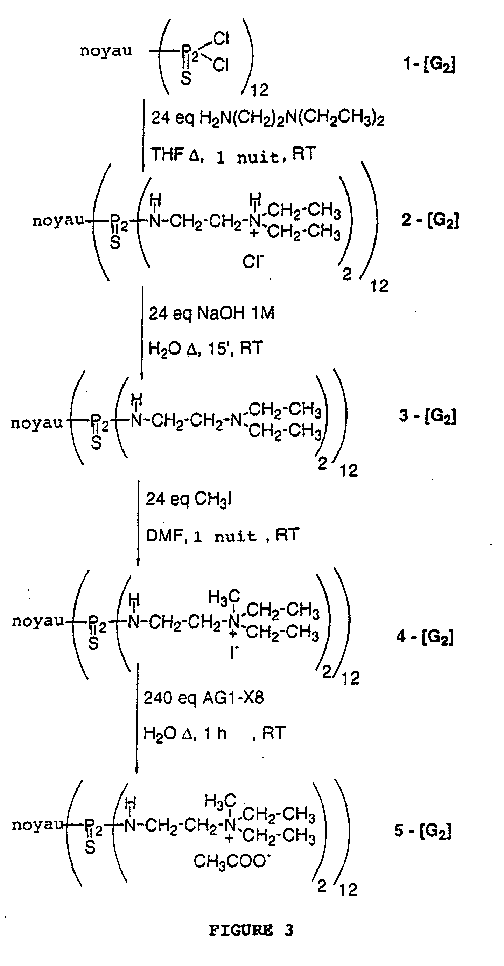

On peut obtenir plusieurs types de produits désignés de manière générale par la formule A-[Gₙ], dans laquelle **A** définit le type d'unité terminale et **G**_{**n**} définit le nombre de couches d'unités intermédiaires P1 (correspondant au nombre de générations) :
- la série des composés 2-[Gₙ] présente des unités terminales de formule III préférée, dans lesquelles X représente un atome d'hydrogène, p est égal à 2, R₃ et R₄ sont identiques et représentent des groupes éthyle, Z est un ion chlorure et n est un nombre entier compris entre 1 et 11, de préférence entre 1 et 10 (voir la figure 3);
- la série des composés 3-[Gₙ] présente des unités terminales de formule III, dans lesquelles X est vide, p est égal à 2, R₃ et R₄ sont identiques et représentent des groupes éthyle, Z est vide et n est un nombre entier compris entre 1 et 11, de préférence entre 1 et 10 (voir la figure 3) ;
- la série des composés 4-[Gₙ] présente des unités terminales de formule III, dans lesquelles X représente un groupe méthyle, p est égal à 2, R₃ et R₄ sont identiques et représentent des groupes éthyle, Z est un ion iodure et n est un nombre entier compris entre 1 et 11, de préférence entre 1 et 10 (voir la figure 3) ;
- la série des composés 5-[Gₙ] présente des unités terminales de formule III, dans lesquelles X représente un groupe méthyle, p est égal à 2, R₃ et R₄ sont identiques et représentent des groupes éthyle, Z est un groupe CH₃COO⁻ et n est un nombre entier compris entre 1 et 11, de préférence entre 1 et 10 (voir la figure 3).
- la série des composés 1-[Gn] présente des unités terminales Cl₂ et n est un nombre entier compris entre 1 et 11, de préférence entre 1 et 10 (voir la figure 3) (produits intermédiaires pour la préparation des dendrimères selon l'invention).

Les dendrimères phosphorés polycationiques selon l'invention présentent un certain nombre d'avantages par rapport aux dendrimères de l'art antérieur utilisés comme vecteurs de gènes :
- ils sont isomoléculaires et sont de ce fait reproductibles (degré de pureté élevé),
- ils sont chargés, ce qui leur confère une affinité particulière vis-à-vis des acides nucléiques à transférer et donc une bonne qualité de transporteur,
- ils sont fonctionnalisables aussi bien en surface qu'au niveau des couches internes,
- ils sont solubles dans l'eau sans dégradation dans une large gamme de pH (3 à 12) (stabilité en solution aqueuse pendant plusieurs mois). Ceci représente un net avantage par rapport aux dendrimères de type PAMAM qui doivent être dégradés thermiquement (étape peu reproductible) pour donner des composés actifs en transfection,
- ils sont peu cytotoxiques (viabilité des cellules transfectées supérieure à 80 %).

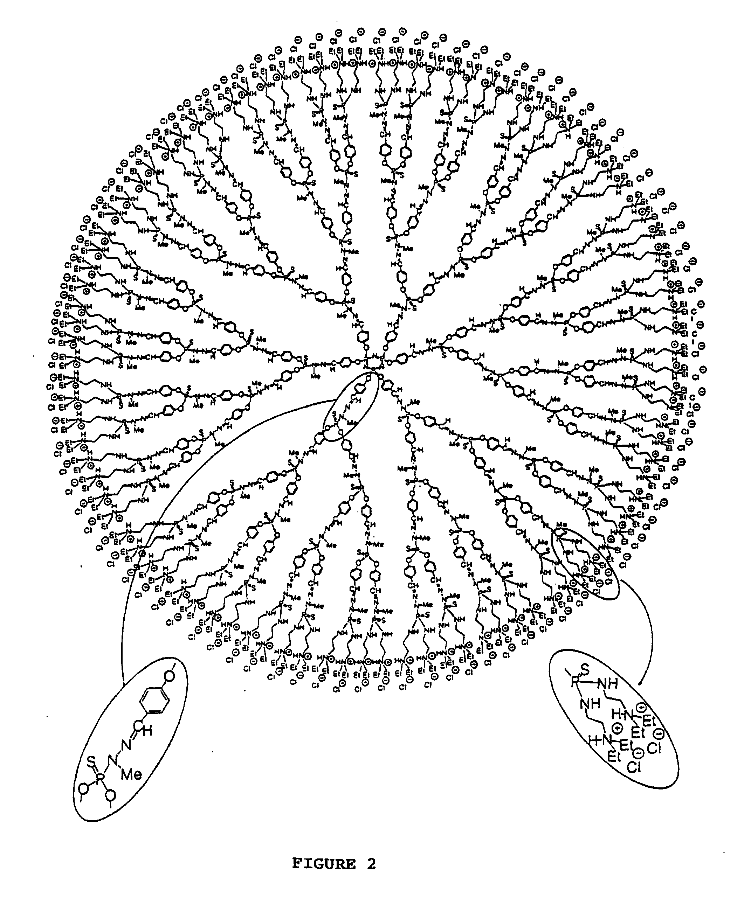

Les dendrimères phosphorés selon l'invention peuvent être obtenus, de manière reproductible, par croissance contrôlée de la structure dendrimérique par ajout de couches successives de motifs ou unités H₂N-N(R₂)-P(S)Cl₂ de formule VIII (exemple de produit de formule II). Le bloc ou noyau central de formule VII (voir figures 1 et 2) correspond à une unité hexachlorocyclotriphosphazène (Cl₆N₃P₃) modifiée par le triéthylammonium de 4-hydroxybenzaldéhyde. Ce noyau, qui comprend 6 fonctions aldéhydes terminales, est mis en contact avec un dichlorophosphonoalkylhydrazide de formule VIII H₂N-N(Alk)-P(S)Cl₂ pour produire un dendron comportant des unités terminales dichlorothiophosphine P(S)Cl₂, qui peuvent être mises en contact à nouveau avec un sel de 4-hydroxybenzaldéhyde de formule VI, pour produire, par itération, chaque génération de dendrimères. Les 1^{ère}, 2^{ème}, 3^{ème}, 4^{ème}, et 5^{ème} générations de dendrimère comprennent respectivement 6, 12, 24, 48 et 96 unités terminales Cl₂, qui peuvent être traitées avec du N,N-dialkyléthylènediamine, pour produire des dendrimères cationiques après protonation : 2-[G₁] à 2-[G₅], conformément à la figure 3.

Ces dendrimères phosphorés polycationiques ont 12, 24, 48, 96 ou 192 charges positives périphériques respectivement pour les 1^{ère}, 2^{ème}, 3^{ème}, 4^{ème}, et 5^{ème} générations de dendrimère.

Les formes méthylées (5-[G₁] à 5-[G₅]) sont préparées à partir des amines terminales neutres correspondantes (3-[G₁] à 3-[G₅]) par une méthylation en présence d'iodure de méthyle, suivie d'un échanges iodures/acétates à l'aide d'une résine convenable (voir exemple 1).

La pureté et l'intégrité des dendrimères est vérifiées par une analyse spectrale (RMN ¹H, ¹³C et ³¹P).

Seulement 8 à 10 % des branches terminales des dendrimères méthylés (5-[G₁] à 5-[G₅]) présentent un déficit en groupes méthyles.

De manière plus précise, les dendrimères selon l'invention peuvent être préparés de la manière suivante :
(1) réaction d'un produit de formule V (aussi dénommé N₃P₃) 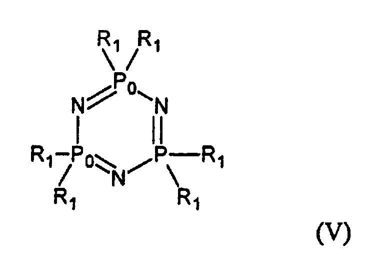 dans lequel R₁ représente un groupement comprenant une fonction aldéhyde, de formule VI : 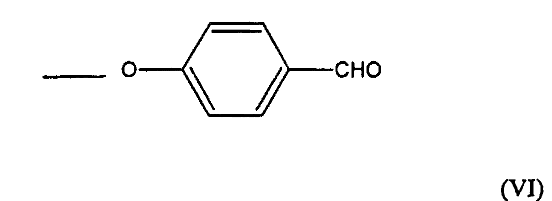 pour obtenir un produit de formule VII: N₃P₃(OC₆H₄CHO)₆ comprenant 6 fonctions aldéhydes (couche centrale constituée d'un noyau P₀ ; figure 3),
(2) réaction du produit de formule VII obtenu en (1) avec un dichlorophosphonohydrazide de formule VIII : H₂N-N(R₂)-P(S)Cl₂ pour produire un dendron de type 1-[Gₙ] comportant des unités terminales Cl₂,
(3) réitération des étapes (1) et (2) sur le produit obtenu en (2) pour produire un nombre n de couches intermédiaires ; les 1^{ère}, 2^{ème}, 3^{ème}, 4^{ème}, et 5^{ème} générations de dendrimère comprennent respectivement 6, 12, 24, 48 et 96 unités terminales Cl₂ et
(4) traitement desdites unités terminales Cl₂ avec du N,N-dialkyléthylènediamine, pour produire des dendrimères cationiques selon l'invention après protonation.

La présente invention a également pour objet une composition capable d'agir comme agent de transfection d'une séquence d'acide nucléique vers une cellule eucaryote, caractérisée en ce qu'elle comprend un acide nucléique et un dendrimère phosphoré polycationique tel que défini ci-dessus, couplé audit acide nucléique.

Selon un mode de réalisation avantageux de ladite composition, elle comprend en outre au moins un véhicule pharmaceutiquement acceptable.

Selon un autre mode de réalisation avantageux de ladite composition, le rapport N/P, dans lequel N correspond aux groupes cationiques terminaux du dendrimère (amines chargées) et P correspond aux groupes phosphates dudit acide nucléique, est compris entre 5 et 10.

Selon un autre mode de réalisation avantageux de ladite composition, elle comprend en outre un agent de perméabilisation de la membrane capable de transporter ledit acide nucléique à travers les membranes cytoplasmiques ou endosomales de ladite cellule eucaryote.

Selon encore un autre mode de réalisation avantageux de ladite composition, ledit dendrimère phosphoré polycationique est associé de manière non-covalente avec ledit acide nucléique.

De manière avantageuse, les dendrimères phosphorés polycationiques selon l'invention, sélectionnés dans la série 2-[Gₙ] dans laquelle n = 3-5 sont particulièrement intéressants comme vecteurs de transfert d'acide nucléique, alors que les dendrimères de la série 5-[Gₙ] sont toxiques et relativement inefficaces dans la transfection d'acides nucléiques dans des cellules eucaryotes aussi bien en l'absence qu'en présence de sérum.

Ce phénomène est peut-être lié à une forte densité en charges positives stables, qui peuvent entraîner l'éclatement de la membrane cellulaire et conduire ainsi à une mort de la cellule. La diminution de la densité de charge n'est pas possible pour les produits alkylés sans dégradation des dendrimères, tandis que la densité de charge de la série 2-[Gn] peut être modulée par des modifications micro-environnementales du pH au niveau de la membrane cellulaire. De plus, la possibilité de moduler la densité de charge de cette série de dendrimères peut constituer un facteur clé dans la libération du gène transporté dans les endosomes. Ces dendrimères agissent ainsi peut-être comme un réservoir de protons dans les compartiments cellulaires, leur densité de charge étant contrôlée par les pompes à protons dépendant de l'ATPase et par des modifications de la concentration intracellulaire en chlorures. La possibilité de réduire la densité de charge cationique de ces dendrimères à l'extérieur et à l'intérieur des cellules devrait être favorables pour leur utilisation *in vivo.*

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la figure 1 illustre la structure du noyau central P₀ (N₃P₃) et un dendrimère selon l'invention de type 3-[G₂] ;
- la figure 2 illustre un dendrimère selon l'invention de type 2-[G₄] ;
- la figure 3 illustre un procédé de préparation des dendrimères selon l'invention ;
- la figure 4 illustre les propriétés de transfert de gène des dendrimères de type 2-[Gₙ], en présence de sérum (parties droites des figures 4A et 4B) ou en l'absence de sérum (parties gauches des figures 4A et 4B) ;
- la figure 5 illustre les propriétés de transfert de gène des dendrimères de type 5-[Gₙ], en présence de sérum (parties droites des figures 5A et 5B) ou en l'absence de sérum (parties gauches des figures 5A et 5B).
   Il doit être bien entendu, toutefois, que ces exemples sont donnés uniquement à titre d'illustration de l'objet de l'invention, dont ils ne constituent en aucune manière une limitation.

### EXEMPLE 1 : Préparation des dendrimères selon l'invention.

### - Méthodes générales et produits utilisés

Toutes les manipulations ont été réalisées par des techniques courantes dans des conditions sous vide poussé ou sous argon. Les spectres RMN du ¹H, du ³¹P et du ¹³C ont été enregistrés sur des spectromètres de la marque Brucker (AC80, AC200 et AC250). Les déplacements chimiques en RMN du ³¹P des réactifs sont exprimées en ppm par rapport à 85% de H₃PO₄. La numérotation utilisée pour la RMN est détaillée sur la figure 1. Les dendrimères 1-[Gₙ] ont été synthétisés selon les protocoles publiés (16, 17). Dans l'abréviation 1-[Gₙ], le chiffre 1 correspond à un dendrimère avec des extrémités Cl, 2, 3, 4 ou 5 pour des extrémités -NH(Et)₂⁺ Cl⁻, -N(Et)₂, -NMe(Et)₂⁺ I⁻ ou -NMe(Et)₂⁺ OAc⁻, respectivement et n correspond au nombre de générations du dendrimère (nombre de couches intermédiaires). L'iodure de méthyle et la N, N-diéthyléthylènediamine ont été fournis par Aldrich et la résine AG1-X8, au fort pouvoir échangeur d'anion, par Biorad.

### - Procédures générales pour la synthèse des produits de type 2-[Gₙ]

De la N,N-diéthyléthylènediamine (n=1, 93µl, 0,66 mmol ; n=2, 71 µl, 0,5 mmol ; n=3, 68 µl, 0,48 mmol ; n=4, 61 µl, 0,43 mmol ; n=5, 60 µl, 0,42 mmol) a été ajoutée goutte à goutte, à la seringue, à une solution contenant 100 mg de dendrimère 1-[Gₙ] (n=1, 55 µmol ; n=2, 21 µmol ; n=3, 10 µmol ; n=4, 4,5 µmol ; n=5, 2,2 µmol) dans 15 ml de THF distillé (THFΔ), sous forte agitation. Après une nuit d'agitation à température ambiante (RT), le solvant a été éliminé par filtration. La poudre blanche obtenue a été lavée deux fois avec 20 ml de THF distillé et séchée par évaporation. Les protons produits pendant la réaction de couplage ont été piégés par les résidus amines tertiaires terminaux, par conséquent les dendrimères 2-[Gₙ] ont été obtenus sous forme de chlorures.

La première génération de dendrimère **2-[G**_{**1**}**]** est obtenue avec un rendement de 80 %. Les données RMN sont les suivantes :
³¹P {¹H} RMN (CD₃OD): δ = 7.9 (P₀), 69.6 (P₁).
¹H RMN (DMSO-*d*₆): δ = 1.3 (t, ³*J*_{HH} = 6.3 Hz, 72 H, CH₂C*H*₃), 3.0-3.5 (m, 114 H, CH₃-N-P₁, CH₂), 5.7 (br m, 12 H, N-H), 7.1 (d, ³*J*_{HH} = 8.4 Hz, 12 H, C₀²-H), 7.9 (s, 6 H, CH=N), 7.9 (d, ³*J*_{HH} = 8.4 Hz, 12 H, C₀³-H), 10.8 (br s, 12 H, ⁺N-H). ¹³C {¹H} RMN (CD₃OD): δ = 9.7 (s, CH₂*C*H₃), 33.3 (d, ²*J*_{CP1} = 10.3 Hz, CH₃-N-P₁), 37.9 (s, CH₂-N-P₁), 49.5 (s, *C*H₂CH₃), 53.9 (d, ³*J*_{CP1} = 6.2 Hz, *C*H₂-CH₂-N-P₁), 122.6 (s, C₀²), 129.8 (s, C₀³), 135.0 (s, C₀⁴), 139.3 (d, ³*J*_{CP1} = 11.6 Hz, CH=N), 152.4 (d, ²*J*_{CP0} = 7.3 Hz, C₀¹).
UV-vis (H₂O): λₘₐₓ (ε, M⁻¹ x cm⁻¹) 284 nm (1.2 x 10⁵).

Deuxième génération de dendrimère **2-[G**_{**2**}**]** (rendement = 95% ) :
³¹P {¹H} RMN (CD₃OD): δ = 8.5 (P₀), 62.0 (P₁), 69.6 (P₂).
¹H RMN (DMSO-*d*₆): δ = 1.3 (br s, 144 H, CH₂C*H*₃), 3.0-3.6 (br m, 246 H, CH₃-N-P_{1,2}, CH₂), 5.6 (br m, 24 H, N-H), 7.0-7.4 (br m, 36 H, C₀²-H, C₁²-H), 7.7-8.2 (m, 54 H, CH=N, C₀³-H, C₁³-H), 10.7 (br s, 24 H, ⁺N-H).
¹³C {¹H} RMN (CD₃OD): δ = 9.6 (s, CH₂CH₃), 33.0 (d, ²*J*_{CP2} = 10.6 Hz, CH₃-N-P₂), 34.2 (d, ²*J*_{CP1} = 11.8 Hz, CH₃-N-P₁), 37.8 (s, CH₂-N-P₂), 49.2 (s, *C*H₂CH₃), 53.9 (d, ³*J*_{CP2} = 6.3 Hz, *C*H₂-CH₂-N-P₂), 122.8 (s, C₀²), 123.0 (d, ³*J*_{CP1} = 3.0 Hz, C₁²), 129.7 (s, C₁³), 130.0 (s, C₀³), 134.3 (s, C₀⁴), 135.0 (s, C₁⁴), 139.1 (d, ³*J*_{CP2} = 12.5 Hz, CH=N), 141.3 (d, ³*J*_{CP1} = 15.4 Hz, CH=N), 152.6 (d, ²*J*_{CP1} = 7.3 Hz, C₁¹), 152.6 (s, C₀¹).
UV-vis (H₂O): λₘₐₓ (ε, M⁻¹ x cm⁻¹) 284 nm (3.1 x 10⁵).

Troisième génération de dendrimère **2-[G**_{**3**}**]** (rendement = 95%) :
³¹P {¹H} RMN (CD₃OD): δ = 8.6 (P₀), 61.5 (P₁), 62.3 (P₂), 69.5 (P₃).
¹H RMN (DMSO-*d*₆): δ = 1.3 (br s, 288 H, CH₂C*H*₃), 3.0-3.5 (br m, 510 H, CH₃-N-P_{1,2,3}, CH₂), 5.7 (br s, 48 H, N-H), 7.0-7.5 (br m, 84 H, C₀²-H, C₁²-H, C₂²-H), 7.7-8.2 (br m, 126 H, CH=N, C₀³-H, C₁³-H, C₂³-H), 10.8 (br s, 48 H, ⁺N-H).
¹³C {¹H} RMN (CD₃OD): δ = 9.6 (s, CH₂*C*H₃), 33.1 (d, ²*J*_{CP3} = 9.4 Hz, CH₃-N-P₃), 34.2 (m, CH₃-N-P_{1,2}), 37.6 (s, CH₂-N-P₃), 49.2 (s, *C*H₂CH₃), 53.7 (d, ³*J*_{CP3} = 6.3 Hz, *C*H₂-CH₂-N-P₃), 123.2 (br s, C₀², C₁², C₂²), 129.6 (br s, C₀³, C₁³, C₂³), 134.0 (s, C₀⁴, C₁⁴), 134.8 (s, C₂⁴), 139.0 (br s, C₂⁴-*C*H=N), 141.4 (br s, CH=N), 152.4 (d, ²*J*_{CP2} = 7.3 Hz, C₂¹), 152.8 (br s, C₀¹, C₁¹).
UV-vis (H₂O): λₘₐₓ (ε, M⁻¹ x cm⁻¹) 286 nm (7.3 x 10⁵).

Quatrième génération de dendrimère **2-[G**_{**4**}**]** (rendement = 95%) :
³¹P {¹H} RMN (CD₃OD): δ = 8.4 (P₀), 62.0 (P_{1,2,3}), 69.4 (P₄).
¹H RMN (DMSO-*d*₆): δ = 1.3 (br s, 576 H, CH₂C*H*₃), 3.0-3.5 (m, 1038 H, CH₃-N-P_{1,2,3,4}, CH₂), 5.6 (br s, 96 H, N-H), 7.0-7.5 (br m, 180 H, C₀²-H, C₁²-H, C₂²-H, C₃²-H), 7.7-8.2 (m, 270 H, CH=N, C₀³-H, C₁³-H, C₂³-H, C₃³-H), 10.8 (br s, 96 H, ⁺N-H).
¹³C {¹H} RMN (CD₃OD): δ = 9.7 (s, CH₂*C*H₃), 33.2 (d, ²*J*_{CP4} = 9.2 Hz, CH₃-N-P₄), 34.3 (d, ²*J*_{CP} = 10.1 Hz, CH₃-N-P_{1,2,3}), 37.7 (s, CH₂-N-P₄), 49.2 (s, *C*H₂CH₃), 53.8 (d, ³*J*_{CP4} = 5.5 Hz, *C*H₂-CH₂-N-P₄), 123.1 (br s, C₀², C₁², C₂², C₃²), 129.7 (br s, C₀³, C₁³, C₂³, C₃³), 134.2 (s, C₀⁴, C₁⁴, C₂⁴), 134.9 (s, C₃⁴), 139.2 (br s, C₃⁴-*C*H=N), 141.5 (br s, CH=N), 152.5 (d, ³*J*_{CP3} = 7.4 Hz, C₃¹), 153.0 (br s, C₀¹, C₁¹, C₂¹).
UV-vis (H₂O): λₘₐₓ (ε, M⁻¹ x cm⁻¹) 288 nm (1.7 x 10⁶).

Cinquième génération de dendrimère **2-[G**_{**5**}**]** (rendement = 95%) :
³¹P {¹H} RMN (CD₃OD): δ = 62.0 (P_{1,2,3,4}), 69.3 (P₅).
¹H RMN (DMSO-*d*₆): δ = 1.3 (br s, 1152 H, CH₂C*H*₃), 2.9-3.5 (br m, 2094 H, CH₃-N-P_{1,2,3,4,5}, CH₂), 5.6 (br s, 192 H, N-H), 7.0-7.5 (m, 372 H, C₀²-H, C₁²-H, C₂²-H, C₃²-H, C₄²-H), 7.7-8.2 (m, 558 H, CH=N, C₀³-H, C₁³-H, C₂³-H, C₃³-H, C₄³-H), 10.8 (br s, 192 H, ⁺N-H).
¹³C {¹H} RMN (CD₃OD): δ = 9.7 (s, CH₂CH₃), 33.2 (br s, CH₃-N-P₅), 34.3 (br s, CH₃-N-P_{1,2,3,4}), 37.8 (s, CH₂-N-P₅), 49.2 (s, *C*H₂CH₃), 53.8 (s, *C*H₂-CH₂-N-P₅), 123.1 (br s, C₀², C₁², C₂², C₃², C₄²), 129.7 (br s, C₀³, C₁³, C₂³, C₃³, C₄³), 134.2 (s, C₀⁴, C₁⁴, C₂⁴, C₃⁴), 134.9 (s, C₄⁴), 139.2 (br s, C₄⁴-CH=N), 141.5 (br s, CH=N), 152.5 (s, C₄¹), 153.0 (br s, C₀¹, C₁¹, C₂¹ , C₃¹).
UV-vis (H₂O): λₘₐₓ (ε, M⁻¹ x cm⁻¹) 286 nm (3.3 x 10⁶).

### - Procédures générales pour la synthèse des produits de type 3-[Gₙ]

Une solution de soude normale (n=1, 372 ml, 0,37 mmol ; n=2, 312 ml, 0,31 mmol ; n=3, 288 ml, 0,29 mmol ; n=4, 288 ml, 0,28 mmol ; n=5, 288 ml, 0,28 mmol) a été ajoutée goutte à goutte à une solution contenant 100 mg de dendrimère 2-[Gₙ] dans 30 ml d'eau distillée (n=1, 31 mmol ; n=2, 13 mmol ; n=3, 6 mmol ; n=4, 3 mmol ; n=5, 1,5 mmol), sous agitation forte. Le précipité a été isolé par centrifugation et dissous dans du chloroforme, puis la couche organique a été séchée sur du sulfate de sodium. Le produit est enfin filtré et séché par évaporation.

Dendrimère **3-[G**_{**1**}**]** (rendement = 80%) :
³¹P {¹H} RMN (CDCl₃): δ = 8.2 (P₀), 68.3 (P₁).
¹H RMN (CD₂Cl₂): δ = 0.9 (t, ³*J*_{HH} = 7.0 Hz, 72 H, CH₂C*H*₃), 2.3-2.5 (m, 72 H, C*H*₂-N(C*H*₂-CH₃)₂), 2.9 (m, 24 H, CH₂-N-P₁), 3.1 (d, ³*J*_{HP1} = 9.4 Hz, 18 H, CH₃-N-P₁), 4.0 (m, 12 H, N-H), 6.9 (d, ³*J*_{HH} = 8.5 Hz, 12 H, C₀²-H), 7.5 (s, 6 H, CH=N), 7.5 (d, ³*J*_{HH} = 8.5 Hz, 12 H, C₀³-H).
¹³C {¹H} RMN (CDCl₃): δ = 11.4 (s, CH₂*C*H₃), 30.5 (d, ²*J*_{CP1} = 10.7 Hz, CH₃-N-P₁), 38.4 (s, CH₂-N-P₁), 46.3 (s, *C*H₂CH₃), 52.9 (d, ³*J*_{CP1} = 7.8 Hz, *C*H₂-CH₂-N-P₁), 120.8 (s, C₀²), 127.3 (s, C₀³), 132.7 (s, C₀⁴), 135.4 (d, ³*J*_{CP1} = 12.5 Hz, CH=N), 150.4 (d, ²*J*_{CP0} = 5.1 Hz, C₀¹).

Dendrimère **3-[G**_{**2**}**]** (rendement = 95%) :
³¹P {¹H} RMN (CDCl₃): δ = 8.4 (P₀), 62.9 (P₁), 68.1 (P₂).
¹H NMR (CD₂Cl₂): δ = 0.9 (t, ³*J*_{HH} = 7 Hz, 144 H, CH₂C*H*₃), 2.2-2.5 (m, 144 H, C*H*₂-N(C*H*₂CH₃)₂), 2.9 (m, 48 H, CH₂-N-P₂), 3.0 (d, ³*J*_{HP2} = 9.2 Hz, 36 H, CH₃-N-P₂), 3.2 (d, ³*J*_{HP1} = 10 Hz, 18 H, CH₃-N-P₁), 4.0 (br m, 24 H, N-H), 6.9-7.1 (m, 36 H, C₀²-H, C₁²-H), 7.4-7.7 (m, 54 H, CH=N, C₀³-H, C₁³-H).
¹³C {¹H} RMN (CDCl₃): δ = 11.4 (s, CH₂*C*H₃), 30.6 (d, ²*J*_{CP2} = 10.8 Hz, CH₃-N-P₂), 32.9 (d, ²*J*_{CP1} = 11.8 Hz, CH₃-N-P₁), 38.3 (s, CH₂-N-P₂), 46.3 (s, *C*H₂CH₃), 52.9 (d, ³*J*_{CP2} = 7.8 Hz, *C*H₂-CH₂-N-P₂), 121.1 (s, C₀², C₁²), 127.4 (s, C₁³), 128.1 (s, C₀³), 132.0 (s, C₀⁴), 133.1 (s, C₁⁴), 135.1 (d, ³*J*_{CP2} = 12.4 Hz, CH=N), 138.6 (d, ³*J*_{CP1} = 15.4 Hz, CH=N), 150.3 (d, ²*J*_{CP1} = 7.3 Hz, C₁¹), 151.1 (s, C₀¹).

Dendrimère **3-[G**_{**3**}**]** (rendement = 95%) :
³¹P {¹H} RMN (CDCl₃): δ = 8.5 (P₀), 62.9 (P_{1,2}), 68.1 (P₃).
¹H RMN (CD₂Cl₂): δ = 0.9 (t, ³*J*_{HH} = 6.4 Hz, 288 H, CH₂C*H*₃), 2.2-2.5 (br m, 288 H, C*H*₂-N(C*H*₂CH₃)₂), 2.9 (br m, 96 H, CH₂-N-P₃), 3.0 (d, ³*J*_{HP3} = 9.2 Hz, 72 H, CH₃-N-P₃), 3.2-3.4 (br m, 54 H, CH₃-N-P_{1,2}), 4.0 (br s, 48 H, N-H), 6.9-7.3 (br m, 84 H, C₀²-H, C₁²-H, C₂²-H), 7.4-7.7 (br m, 126 H, CH=N, C₀³-H, C₁³-H, C₂³-H). ¹³C {¹H} RMN (CDCl₃): δ = 11.4 (s, CH₂*C*H₃), 30.6 (d, ²*J*_{CP3} = 10.3 Hz, CH₃-N-P₃), 32.5 (d, ²*J*_{CP} = 12.7 Hz, CH₃-N-P_{1,2}), 38.4 (s, CH₂-N-P₃), 46.4 (s, *C*H₂CH₃), 53.0 (d, ³*J*_{CP3} = 7.9 Hz, *C*H₂-CH₂-N-P₃), 121.4 (s, C₀², C₁², C₂²), 127.5 (s, C₂³), 128.2 (s, C₀³, C₁³), 132.0 (s, C₀⁴), 132.3 (s, C₁⁴), 133.1 (s, C₂⁴), 135.2 (d, ³*J*_{CP3} = 12.1 Hz, CH=N), 138.8 (d, ³*J*_{CP} = 12.1 Hz, CH=N), 150.4 (d, ²*J*_{CP2} = 6.8 Hz, C₂¹), 151.2 (d, ²*J*_{CP} = 6.3 Hz, C₀¹, C₁¹).

Dendrimère **3-[G**_{**4**}**]** (rendement = 95%) :
³¹P {¹H} RMN (CDCl₃): δ = 8.4 (P₀), 62.4 (P_{1,2,3}), 68.0 (P₄).
¹H RMN (CD₂Cl₂): δ = 0.8 (br s, 576 H, CH₂C*H*₃), 2.4 (br s, 576 H, C*H*₂-N(C*H*₂CH₃)₂), 2.6-3.4 (br m, 462 H, CH₃-N-P_{1,2,3,4}, CH₂-N-P₄), 4.0 (br m, 96 H, N-H), 7.0-7.7 (m, 450 H, C₆H₄, CH=N).
¹³C {¹H} RMN (CDCl₃): δ = 11.6 (s, CH₂CH₃), 30.6 (d, ²*J*_{CP4} = 10.4 Hz, CH₃-N-P₄), 33.0 (d, ²*J*_{CP} = 12.6 Hz, CH₃-N-P_{1,2,3}), 38.5 (s, CH₂-N-P₄), 46.4 (s, CH₂CH₃), 53.1 (d, ³*J*_{CP4} = 7.7 Hz, *C*H₂-CH₂-N-P₄), 121.5 (s, C₃²), 121.8 (s, C₀², C₁², C₂²), 127.5 (s, C₃³), 128.3 (s, C₀³, C₁³, C₂³), 132.2 (s, C₀⁴, C₁⁴, C₂⁴), 133.2 (s, C₃⁴), 135.1 (d, ³*J*_{CP4} = 12.0 Hz, CH=N), 138.7 (br s, CH=N), 150.5 (d, ²*J*_{CP2} = 7.4 Hz, C₃¹), 151.3 (br m, C₀¹, C₁¹, C₂¹).

Dendrimère **3-[G**_{**5**}**]** (rendement = 95%) :
³¹P {¹H} RMN (CDCl₃): δ = 62.4 (P_{1,2,3,4}), 68.0 (P₅).
¹H NMR (CD₂Cl₂): δ = 1.0 (br s, 1152 H, CH₂C*H*₃), 2.4 (br s, 1152 H, C*H*₂-N(C*H*₂CH₃)₂), 2.7-3.5 (br m, 942 H, CH₃-N-P_{1,2,3,4,5}, CH₂-N-P₅), 4.1 (m, 192 H, N-H), 7.0-7.8 (m, 930 H, C₆H₄, CH=N).
¹³C {¹H} RMN (CDCl₃): δ = 11.5 (s, CH₂*C*H₃), 30.5 (d, ²*J*_{CP5} = 11.1 Hz, CH₃-N-P₅), 33.0 (d, ²*J*_{CP} = 12.3 Hz, CH₃-N-P_{1,2,3,4}), 38.4 (s, CH₂-N-P₅), 49.4 (s, *C*H₂CH₃), 53.0 (d, ³*J*_{CP5} = 8.1 Hz, *C*H₂-CH₂-N-P₅), 121.4 (s, C₄²), 121.7 (s, C₀², C₁², C₂^{2,}, C₃²), 127.4 (s, C₄³), 128.1 (s, C₀³, C₁³, C₂³, C₃³), 132.1 (s, C₀⁴, C₁⁴, C₂⁴, C₃⁴), 133.1 (s, C₄⁴), 135.1 (d, ³*J*_{CP5} = 12.1 Hz, CH=N), 138.7 (br s, CH=N), 150.5 (d, ²*J*_{CP3} = 6.6 Hz, C₄¹), 151.2 (d, ²*J*_{CP} = 6.2 Hz, C₀¹, C₁¹, C₂¹, C₃¹).

### - Procédures générales pour la synthèse des produits de type 4-[Gₙ]

Une solution comprenant 100 mg de dendrimères neutres 3-[Gₙ] (n=1, 36 mmol ; n=2, 15 mmol ; n=3, 7 mmol ; n=4, 3,3 mmol ; n=5, 1,6 mmol) et de l'iodure de méthyle (n=1, 27 µl, 0,43 mmol ; n=2, 22 µl, 0,36 mmol; n=3, 21 µl, 0,34 mmol ; n=4, 20 µl, 0,32 mmol ; n=5, 19 µl, 0,31 mmol) dans 15 ml de DMF a été agitée toute la nuit à température ambiante. La solution a été séchée par évaporation. La pâte obtenue a été lavée avec 20 ml d'un mélange pentane/éther (1/1, v/v) pour obtenir une poudre jaune de dendrimères méthylés dénommés 4-[Gₙ].

Dendrimère **4-[G**_{**1**}**]** (rendement = 90%):
³¹P {¹H} RMN (DMSO-*d*₆): δ = 7.3 (P₀), 68.1 (P₁).
¹H RMN (DMSO-*d*₆): δ = 1.3 (t, ³*J*_{HH} = 6.4 Hz, 72 H, CH₂C*H*₃), 3.1 (s, 36 H, ⁺N-CH₃), 3.2 (d, ³*J*_{HP1} = 10.4 Hz, 18 H, CH₃-N-P₁), 3.3-3.7 (br s, 96 H, CH₂), 5.5 (br m, 12 H, N-H), 7.1 (d, ³*J*_{HH} = 8.1 Hz, 12 H, C₀²-H), 8.0 (s, 6 H, CH=N), 8.0 (d, ³*J*_{HH} = 8.1 Hz, 12 H, C₀³-H).
¹³C {¹H} RMN (DMSO-*d*₆): δ = 7.8 (s, CH₂*C*H₃), 32.6 (d, ²*J*_{CP1} = 9.5 Hz, CH₃-N-P₁), 34.9 (s, CH₂-N-P₁), 47.3 (s, ⁺N-CH₃), 56.4 (s, *C*H₂CH₃), 58.8 (s, *C*H₂-CH₂-N-P₁), 120.7 (s, C₀²), 128.3 (s, C₀³), 133.2 (s, C₀⁴), 137.4 (d, ³*J*_{CP1} = 14.2 Hz, CH=N), 149.9 (s, C₀¹).

Dendrimère **4-[G**_{**2**}**]** (rendement quantitatif) :
³¹P {¹H) RMN (DMSO-*d*₆): δ = 7.3 (P₀), 61.7 (P₁), 68.1 (P₂).
¹H RMN (DMSO-*d*₆): δ = 1.3 (t, ³*J*_{HH} = 6.2 Hz, 144 H, CH₂C*H*₃), 3.1 (s, 72 H, ⁺N-CH₃), 3.2-3.6 (m, 246 H, CH₃-N-P_{1,2}, CH₂), 5.7 (br s, 24 H, N-H), 7.0-7.4 (m, 36 H, C₀²-H, C₁²-H), 7.7-8.2 (m, 54 H, CH=N, C₀³-H, C₁³-H).
¹³C {¹H} RMN (DMSO-*d*₆): δ = 7.8 (s, CH₂*C*H₃), 32.2 (d, ²*J*_{CP2} = 8.9 Hz, CH₃-N-P₂), 33.5 (d, ²*J*_{CP1} = 12.9 Hz, CH₃-N-P₁), 34.9 (s, CH₂-N-P₂), 47.3 (s, ⁺N-CH₃), 56.4 (s, *C*H₂CH₃), 58.8 (d, ³*J*_{CP2} = 3.3 Hz, *C*H₂-CH₂-N-P₂), 121.3 (s, C₁², C₀²), 128.2 (s, C₁³), 128.6 (s, C₀³), 132.3 (s, C₀⁴), 133.2 (s, C₁⁴), 137.1 (d, ³*J*_{CP2} = 12.3 Hz, CH=N), 140.7 (br s, CH=N), 150.1 (d, ²*J*_{CP1} = 6 Hz, C₁¹), 150.5 (s, C₀¹).

Dendrimère **4-[G**_{**3**}**]** (rendement quantitatif):
³¹P {¹H} RMN (DMSO-*d*₆): δ = 6.9 (P₀), 61.9 (P_{1,2}), 68.1 (P₃).
¹H RMN (DMSO-*d*₆): δ = 1.3 (br s, 288 H, CH₂C*H*₃), 3.1 (s, 144 H, ⁺N-CH₃), 3.1-3.6 (m, 510 H, CH₃-N-P_{1,2,3}, CH₂), 5.5 (br s, 48 H, N-H), 7.0-7.4 (m, 84 H, C₀²-H, C₁²-H, C₂²-H), 7.7-8.2 (m, 126 H, CH=N, C₀³-H, C₁³-H, C₂³-H).
¹³C {¹H} RMN (DMSO-*d*₆): δ = 7.7 (s, CH₂*C*H₃), 32.2 (d, ²*J*_{CP3} = 9.4 Hz, CH₃-N-P₃), 33.5 (m, CH₃-N-P_{1,2}), 34.9 (s, CH₂-N-P₃), 47.3 (s, ⁺N-CH₃), 56.4 (s, *C*H₂CH₃), 58.8 (d, ³*J*_{CP3} = 4.8 Hz, *C*H₂-CH₂-N-P₃), 121.0 (br s, C₀², C₁², C₂²), 128.5 (br s, C₀³, C₁³, C₂³), 132.3 (s, C₀⁴, C₁⁴), 133.2 (s, C₂⁴), 137.1 (d, ³*J*_{CP3} = 12.1 Hz, CH=N), 141.0 (br s, CH=N), 150.1 (d, ²*J*_{CP2} = 6.2 Hz, C₂¹), 150.7 (br s, C₀¹, C₁¹).

Dendrimère **4-[G**_{**4**}**]** (rendement quantitatif) :
³¹P {¹H} RMN (DMSO-*d*₆): δ = 6.3 (P₀), 61.6 (P_{1,2,3}), 68.0 (P₄).
¹H RMN (DMSO-*d*₆): δ = 1.3 (br s, 576 H, CH₂CH₃), 3.1 (s, 288 H, ⁺N-CH₃), 3.1-3.6 (m, 1038 H, CH₃-N-P_{1,2,3,4}, CH₂), 5.5 (br s, 96 H, N-H), 7.1-8.5 (m, 450 H, C₆H₄, CH=N).
¹³C {¹H} RMN (DMSO-*d*₆): δ = 7.8 (s, CH₂*C*H₃), 32.2 (d, ²*J*_{CP4} = 9.5 Hz, CH₃-N-P₄), 33.6 (d, ²*J*_{CP} = 7.6 Hz, CH₃-N-P_{1,2,3}), 34.9 (s, CH₂-N-P₄), 47.3 (s, ⁺N-CH₃), 56.4 (s, *C*H₂CH₃), 58.7 (d, ³*J*_{CP4} = 4.8 Hz, *C*H₂-CH₂-N-P₄), 121.2 (br s, C₀², C₁², C₂², C₃²), 128.2 (br s, C₀³, C₁³, C₂³ , C₃³), 132.2 (s, C₀⁴, C₁⁴, C₂⁴), 133.2 (s, C₃⁴), 137.1 (d, ³*J*_{CP4} = 9.4 Hz, CH=N), 140.8 (br s, CH=N), 150.0 (d, ²*J*_{CP2} = 6.2 Hz, C₃¹), 150.7 (br s, C₀¹, C₁¹, C₂¹).

Dendrimère **4-[G**_{**5**}**]** (rendement quantitatif) :
³¹P {¹H} RMN (DMSO-*d*₆): δ = 61.6 (P_{1,2,3,4}), 68.0 (P₅).
¹H RMN (DMSO-*d*₆): δ = 1.3 (br s, 1152 H, CH₂C*H*₃), 3.1 (s, 576 H, ⁺N-CH₃), 3.1-3.6 (m, 2094 H, CH₃-N-P_{1,2,3,4,5}, CH₂), 5.4 (m, 192 H, N-H), 7.2-8.5 (m, 930 H, C₆H₄ , CH=N).
¹³C {¹H} RMN (DMSO-*d*₆): δ = 7.8 (s, CH₂*C*H₃), 32.3 (br s, CH₃-N-P₅), 33.5 (br s, CH₃-N-P_{1,2,3,4}), 34.9 (s, CH₂-N-P₅), 47.3 (s, ⁺N-CH₃), 56.4 (s, *C*H₂CH₃), 58.8 (d, ³*J*_{CP} = 5.0 Hz, *C*H₂-CH₂-N-P₅), 121.3 (br s, C₀², C₁², C₂², C₃², C₄²), 128.2 (br s, C₀³, C₁³, C₂³, C₃³, C₄³), 132.2 (s, C₀⁴, C₁⁴, C₂⁴, C₃⁴), 133.2 (s, C₄⁴), 137.2 (d, ³*J*_{CP4} = 9.7 Hz, CH=N), 141.2 (br s, CH=N), 150.1 (d, ²*J*_{CP2} = 6.0 Hz, C₄¹), 150.7 (br s, C₀¹, C₁¹, C₂¹, C₃¹).

### - Procédures générales pour la synthèse des produits de type 5-[Gₙ]

La résine à fort pouvoir échangeur d'anions AG1-X8 a été ajoutée à une suspension de 100 mg de dendrimères méthylés 4-[Gₙ], (sous forme d'iodures), (n=1, 22 µmol ; n=2, 10 µmol ; n=3, 4,7 µmol ; n=4, 21 µmol ; n=5, 1,1 µmol) dans l'eau distillée (n=1, 25 ml ; n=2, 23 ml ; n=3, 22 ml ; n=4, 21 ml ; n=5 ; 20 ml), dans les proportions indiquées (n=1, 1,24 g ; n=2, 1,13 g ; n=3, 1,06 g ; n=4, 1,03 g ; n=5, 1 g) et mélangée doucement pendant une heure. La pâte obtenue a été lavée avec 20 ml d'un mélange pentane/ether (1/1, v/v) pour obtenir une poudre blanche de dendrimères méthylés, sous forme acétate, dénommés **5-[G**_{**n**}**]**. 8 à 10 % des ramifications terminales sont modifiées lors de l'échange de contre-ions avec la résine et ont été provisoirement attribués à une forme déméthylée puisque les résultats de RMN étaient identiques à ceux des dendrimères neutres portant des amines tertiaires (3-[Gₙ]. Ces ramifications terminales mineures ont été indiquées par une astérisque dans les résultats de RMN présentés ci-dessous et les rendements correspondent à ceux du dendrimère total.

Dendrimère **5-[G**_{**1**}**]** (rendement = 90%) :
³¹P{¹H} RMN (CD₃OD): δ = 7.8 (P₀), 69.8 (P₁*), 70.3 (P₁).
¹H RMN (DMSO-*d*₆): δ = 1.0 (t, ³*J*_{HH} = 6.9 Hz, 6 H, CH₂C*H*₃*), 1.3 (t, ³*J*_{HH} = 6.5 Hz, 66 H, CH₂C*H*₃), 1.7 (s, 33 H, CH₃COO⁻), 2.5 (m, 6 H, C*H*₂*-N(C*H*₂*CH₃)₂), 3.1 (s, 33 H, ⁺N-CH₃), 3.2 (d, ³*J*_{HP1} = 8.3 Hz, 18 H, CH₃-N-P₁), 3.3-3.7 (m, 90 H, CH₂), 7.1 (d, ³*J*_{HH} = 8.3 Hz, 12 H, C₀²-H), 7.6 (b, 12 H, N-H), 7.8 (s, 6 H, CH=N), 7.8 (d, ³*J*_{HH} = 8.3 Hz, 12 H, C₀³-H).
¹³C {¹H} RMN (DMSO-*d*₆): δ = 7.6 (s, CH₂*C*H₃), 11.9 (s, CH₂*C**H₃), 25.6 (s, *C*H₃COO⁻), 32.2 (d, ²*J*_{CP1} = 9.5 Hz, CH₃-N-P₁), 38.8 (s, CH₂-N-P₁), 46.6 (s, *C**H₂CH₃), 47.0 (s, ⁺N-CH₃), 54.5 (s, *C**H₂-CH₂-N-P₁), 56.2 (s, *C*H₂CH₃), 59.1 (d, ³*J*_{CP1} = 5.3 Hz, *C*H₂-CH₂-N-P₁), 120.6 (s, C₀²), 127.9 (s, C₀³), 133.7 (s, C₀⁴), 135.8 (d, ²*J*_{CP0} = 10.8 Hz, CH=N), 149.8 (s, C₀¹), 173.7 (s, CH₃*C*OO⁻).
UV-vis (H₂O): λₘₐₓ (ε, M⁻¹ x cm⁻¹) 284 nm (1.2 x 10⁵).

Dendrimère **5-[G**_{**2**}**]** (rendement = 95%) :
³¹P {¹H} RMN (CD₃OD): δ = 8.4 (P₀), 62.3 (P₁), 69.5 (P₂*), 70.1 (P₂).
¹H NMR (DMSO-*d*₆): δ = 1.0 (br t, 12 H, CH₂C*H*₃*), 1.3 (br s, 132 H, CH₂C*H*₃), 1.7 (s, 66 H, CH₃COO⁻), 2.5 (br m, 12 H, C*H*₂*-N(C*H*₂*CH₃)₂), 3.1 (s, 66 H, ⁺N-CH₃), 3.2-3.6 (m, 234 H, CH₃-N-P_{1,2}, CH₂), 7.1-7.3 (br m, 36 H, C₀²-H, C₁²-H), 7.6 (br s, 24 H, N-H), 7.7 (s, 12 H, CH=N), 7.8-8.1 (br m, 42 H, CH=N, C₀³-H, C₁³-H).
¹³C {¹H} RMN (DMSO-*d*₆): δ = 7.6 (s, CH₂*C*H₃), 11.9 (s, CH₂*C**H₃), 25.6 (s, *C*H₃COO⁻), 32.1 (d, ²*J*_{CP2} = 9.3 Hz, CH₃-N-P₂), 33.2 (d, ²*J*_{CP1} = 13.4 Hz, CH₃-N-P₁), 35.0 (s, CH₂-N-P₂), 46.6 (s, *C**H₂CH₃), 47.0 (s, ⁺N-CH₃), 54.5 (s, *C**H₂-CH₂-N-P₂), 56.2 (s, CH₂CH₃), 59.1 (d, ³*J*_{CP2} = 5.3 Hz, *C*H₂-CH₂-N-P₂), 121.3 (s, C₀², C₁²), 127.9 (s, C₁³), 128.5 (s, C₀³), 132.3 (s, C₀⁴), 133.8 (s, C₁⁴), 135.7 (br s, CH=N), 149.8 (d, ²*J*_{CP1} = 6.5 Hz, C₁¹), 150.7 (s, C₀¹), 173.7 (s, CH₃*C*OO⁻).
UV-vis (H₂O): λₘₐₓ (ε, M⁻¹ x cm⁻¹) 284 nm (4.1 x 10⁵).

Dendrimère **5-[G**_{**3**}**]** (rendement = 95%) :
³¹P {¹H} RMN (CD₃OD): δ = 8.4 (P₀), 62.3 (P_{1,2}), 69.5 (P₃*), 70.1 (P₃).
¹H RMN (DMSO-*d*₆): δ = 1.0 (br t, 30 H, CH₂C*H*₃*), 1.3 (br s, 258 H, CH₂C*H*₃), 1.7 (s, 129 H, CH₃COO⁻), 2.5 (br m, 30 H, C*H*₂*-N(C*H*₂*CH₃)₂), 3.1 (s, 129 H, ⁺N-CH₃), 3.1-3.6 (m, 480 H, CH₃-N-P_{1,2,3}, CH₂), 7.1-7.5 (br m, 108 H, C₀²-H, C₁²-H, C₂²-H, N-H), 7.5-8.1 (br m, 126 H, CH=N, C₀³-H, C₁³-H, C₂³-H).
¹³C {¹H} RMN (DMSO-*d*₆): δ = 7.6 (s, CH₂*C*H₃), 11.9 (s, CH₂*C**H₃), 25.2 (s, *C*H₃COO⁻), 32.1 (d, ²*J*_{CP3} = 9.0 Hz, CH₃-N-P₃), 33.2 (br m, CH₃-N-P_{1,2}), 35.0 (s, CH₂-N-P₃), 46.6 (s, *C**H₂CH₃), 47.1 (s, ⁺N-CH₃), 54.5 (s, *C**H₂-CH₂-N-P₃), 56.3 (s, *C*H₂CH₃), 59.1 (d, ³*J*_{CP3} = 5.7 Hz, *C*H₂-CH₂-N-P₃), 121.3 (br s, C₀², C₁², C₂²), 128.0 (s, C₂³), 128.5 (s, C₀³, C₁³), 132.4 (s, C₀⁴, C₁⁴), 133.8 (s, C₂⁴), 136.3 (br d, C₂⁴-CH=N), 141.0 (br m, CH=N), 150.1 (d, ²*J*_{CP2} = 6.3 Hz, C₂¹), 151.0 (d, ²*J*_{CP} = 7.1 Hz, C₀¹, C₁¹), 173.7 (s, CH₃COO⁻).
UV-vis (H₂O): λₘₐₓ (ε, M⁻¹ x cm⁻¹) 286 nm (7.4 x 10⁵).

Dendrimère **5-[G**_{**4**}**]** (rendement = 95%) :
³¹P {¹H} RMN (CD₃OD): δ = 8.2 (P₀), 62.2 (P_{1,2,3}), 69.9 (P₄).
¹H RMN (DMSO-*d*₆): δ = 1.0 (br m, 60 H, CH₂C*H*₃*), 1.3 (br s, 516 H, CH₂C*H*₃), 1.7 (s, 258 H, CH₃COO⁻), 2.5 (m, 60 H, C*H*₂*-N(C*H*₂*CH₃)₂), 3.1 (s, 258 H, ⁺N-CH₃), 3.1-3.6 (m, 978 H, CH₃-N-P_{1,2,3,4}, CH₂), 7.0-8.2 (m, 546 H, C₆H₄, CH=N, N-H).
¹³C {¹H} RMN (DMSO-*d*₆): δ = 7.6 (s, CH₂*C*H₃), 11.9 (s, CH₂*C**H₃), 24.7 (s, *C*H₃COO⁻), 32.1 (d, ²*J*_{CP4} = 9.6 Hz, CH₃-N-P₄), 33.2 (d, ²*J*_{CP} = 11.2 Hz, CH₃-N-P_{1,2,3}), 35.0 (s, CH₂-N-P₄), 46.6 (s, *C**H₂CH₃), 47.0 (s, ⁺N-CH₃), 54.5 (d, ³*J*_{CP4} = 3.9 Hz, *C**H₂-CH₂-N-P₄), 56.3 (s, *C*H₂CH₃), 59.2 (d, ³*J*_{CP4} = 5.7 Hz, *C*H₂-CH₂-N-P₄), 121.2 (s, C₃²), 121.6 (s, C₀², C₁², C₂²), 127.9 (s, C₃³), 128.5 (s, C₀³, C₁³, C₂³), 132.3 (s, C₀⁴, C₁⁴, C₂⁴), 133.7 (s, C₃⁴), 135.8 (br s, C₃⁴-CH=N), 141.0 (br s, CH=N), 149.9 (d, ²*J*_{CP3} = 6.3 Hz, C₃¹), 151.0 (br m, C₀¹,C₁¹, C₂¹), 173.7 (s, CH₃*C*OO⁻).
UV-vis (H₂O): λₘₐₓ (ε, M⁻¹ x cm⁻¹) 288 nm (1.6 x 10⁶).

Dendrimère **5-[G**_{**5**}**]** (rendement = 95%) :
³¹P {¹H} RMN (DMSO-*d*₆): δ = 62.2 (P_{1,2,3,4}), 69.9 (P₅).
¹H RMN (DMSO-*d*₆): δ = 1.0 (m, 114 H, CH₂C*H*₃*), 1.3 (m, 1038 H, CH₂C*H*₃), 1.7 (s, 519 H, CH₃COO⁻), 2.5 (m, 114 H, C*H*₂*-N(C*H*₂*CH₃)₂), 3.1 (s, 519 H, ⁺N-CH₃), 3.1-3.6 (m, 1980 H, CH₃-N-P_{1,2,3,4,5}, CH₂), 7.0-8.2 (m, 1122 H, C₆H₄, CH=N, N-H).
¹³C {¹H} RMN (DMSO-*d*₆): δ = 7.6 (s, CH₂CH₃), 11.9 (s, CH₂*C**H₃), 24.7 (s, *C*H₃COO⁻), 32.1 (d, ²*J*_{CP5} = 9.6 Hz, CH₃-N-P₅), 33.2 (d, ²*J*_{CP} = 11.2 Hz, CH₃-N-P_{1,2,3,4}), 35.0 (s, CH₂-N-P₅), 46.6 (s, *C**H₂CH₃), 47.0 (s, ⁺N-CH₃), 54.5 (d, ³*J*_{CP5} = 3.9 Hz, *C**H₂-CH₂-N-P₅), 56.3 (s, *C*H₂CH₃), 59.2 (d, ³*J*_{CP5} = 5.7 Hz, *C*H₂-CH₂-N-P₅), 121.2 (s, C₀², C₁², C₂², C₃², C₄²), 127.9 (s, C₄³), 128.5 (s, C₀³, C₁³, C₂³, C₃³), 132.3 (s, C₀⁴, C₁⁴, C₂⁴, C₃⁴), 133.7 (s, C₄⁴), 135.9 (br s, C₄⁴-*C*H=N), 141.0 (br s, CH=N), 149.9 (d, ²*J*_{CP3} = 6.5 Hz, C₄¹), 151.0 (br m, C₀¹, C₁¹, C₂¹, C₃¹), 173.7 (s, CH₃COO⁻).
UV-vis (H₂O): λₘₐₓ (ε, M⁻¹ x cm⁻¹) 284 nm (3.7 x 10⁶).

### EXEMPLE 2 : Expériences de transfection.

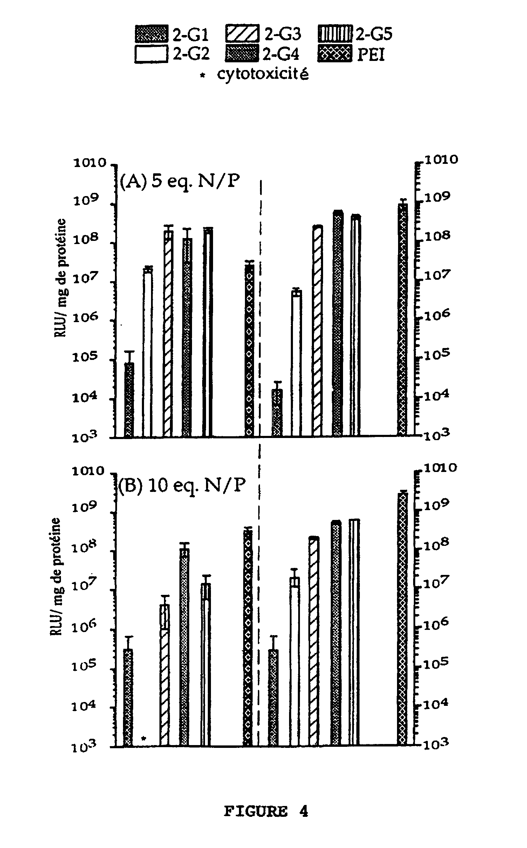

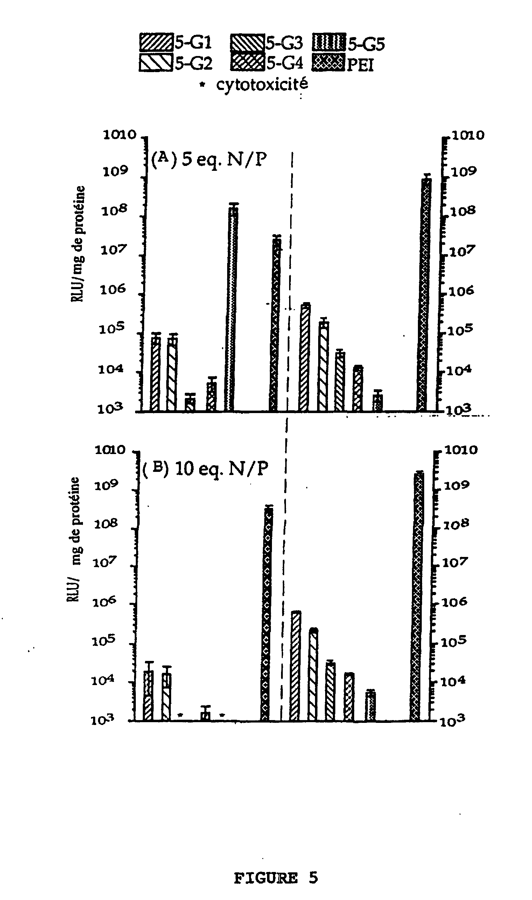

Les résultats obtenus avec ces différents dendrimères phosphorés polycationiques ont toujours montré une efficacité optimale avec un rapport de charges N/P compris entre 5 (figure 4) et 10 (figure 5) (rapport N/P=nombre d'atomes d'azote terminaux du dendrimère par phosphate de l'ADN). Ainsi, il a été décidé, de façon arbitraire, de comparer l'efficacité de transfection de différentes générations de dendrimères phosphorés (P-dendrimères) avec celle du PEI linéaire ExGen 500, possédant 5 à 10 équivalents amines par nucléotide.

Avec 5 équivalents de charges positives par phosphate de l'ADN, les 5 différentes générations de dendrimères protonés de type 2-[Gₙ], testés sous forme de chlorhydrates, ont permis d'obtenir une expression significative du transgène. Une augmentation de 10⁵ à presque 10⁹ unités relatives de luminescence (RLU)/mg de protéine a été observée. L'efficacité de transfection augmente avec la taille du dendrimère (nombre de génération) mais atteint un plateau à partir de la troisième génération, avec des valeurs comprises entre 10⁸ et 10⁹ RLU (Fig. 4). Par conséquent, 2-[G₄] a été choisi pour étudier plus en détail l'efficacité de transfection de ces nouveaux dendrimères cationiques phosphorés, dont la structure est présentée sur la figure 2.

Il faut remarquer que les transfections effectuées en présence de sérum conduisent à une plus faible toxicité. De ce fait, un niveau d'expression plus élevé est observé pour les transfections effectuées en présence de sérum par rapport à celles effectuées sans sérum (Fig. 4A et 4B), panneaux droits et gauches, respectivement).

Sans essayer d'optimiser davantage leurs conditions de transfection, les dendrimères de la troisième à la cinquième génération se sont révélés aussi efficaces que le PEI linéaire utilisé en conditions optimales.

En revanche, les formes méthylées 5-[Gₙ] se sont montrées plutôt toxiques et peu efficaces pour transfecter des acides nucléiques dans des cellules eucaryotes (Fig 5A et 5B). Ce phénomène peut s'expliquer du fait d'une densité de charge positive stable qui peut perturber la membrane cellulaire et provoquer la mort cellulaire. La diminution de la densité de charge des formes méthylées n'est pas réalisable sans dégrader le dendrimère, en revanche la densité de charge des dérivés chlorures (2-[Gₙ]) peut être modulée par des modifications micro-environnementales du pH lorsqu'ils approchent la membrane cellulaire. De plus, la possibilité de moduler la densité de charge des dérivés chlorures des dendrimères pourrait jouer un rôle clé dans le relargage du gène luciférase à partir de l'endosome. Ces dendrimères se comportent peut-être comme des réservoirs à protons dans les compartiments cellulaires, leur densité de charge étant contrôlée d'une part par des pompes à protons couplées à des ATPases, et d'autre part par des modifications intracellulaires de la concentration en chlorures. La possibilité de réduire la densité de charge cationique de ces dendrimères phosphorés, à l'intérieur ou à l'extérieur des cellules, serait un avantage pour la réalisation d'expériences *in vivo,* comme cité précédemment (26).

### - Produits chimiques

Le PEI linéaire de 22 kDa (ExGen 500) a été fourni par Euromedex (Souffelweyersheim, France).

### - Lignées et culture cellulaires.

Les fibroblastes murins NIH3T proviennent de l'ATCC (Rockville, MA, USA) et sont cultivés en milieu Dulbecco modifié par Eagle (DMEM). Les milieux de culture sont supplémentés avec 10 % de sérum de veau foetal (D. Dutscher, Brunath, France), 2 mM de L-glutamine (Gibco-BRL), 100 unités/ml de pénicilline (Gibco-BRL) et 100 µg/ml de streptomycine (Gibco-BRL). Les cellules sont maintenues à 37°C, en atmosphère humide contenant 5 % de CO₂ . Lorsque les cellules atteignent 80 % de confluence, elles sont décollées par une solution saline de trypsine-EDTA (Gibco-BRL), diluées d'un facteur dix et cultivées dans un nouveau flacon.

### - Plasmides

PCMV-luc, codant pour la luciférase de *Photinus pyralis*, sous le contrôle des séquences promoteur/ehhanceur du cytomégalovirus ont été gracieusement fournies par le Dr. M. Scarpa (CRIBI, padoue, Italie). Les plasmides ont été purifiés sur des colonnes Qiagen (Rockford, USA) à partir de la souche XL1 d'*E. Coli* transformée.

### - Transfection des cellules

Les cellules adhérentes ont été ensemencées dans des plaques à 24 puits (Costar, D. Dutscher, France), la veille de la transfection, de façon à atteindre 60 à 70 % de confluence le jour de la transfection. Toutes les expériences ont été réalisées en triple. Les cellules ont été rincées avant la transfection et 1 ml de milieu avec ou sans sérum a été ajouté dans chaque puits. 2 µg de plasmide (solution à 1,5 mg/ml dans un tampon 10 mM Tris-1 mM EDTA, pH 7,4) ont été dilués dans 50 µl de NaCl 0,15 M.

Le rapport N/P (azote/phosphate) correspond à la quantité de polymère nécessaire pour avoir un résidu aminé (43Da=poids moléculaire moyen (M_{w}) pour le PEI ; pour les dendrimères la molarité en azote des résidus amines a été calculée en divisant le M_{w} par le nombre de charges pour chaque génération) par phosphate d'acide nucléique (M_{w}330) (12). La quantité nécessaire de PEI linéaire (ExGen500) et de dendrimères (à partir de solutions stock de PEI et de dendrimères correspondant à 10 mM d'azote, sous forme amine, dans l'eau MilliQ stérile) a été diluée dans 50 µl de NaCl 0,15 M, vortexée doucement et centrifugée. Après 15 min, le vecteur cationique a été ajouté, en une seule fois, à la solution de plasmide [et pas dans l'ordre inverse, (12)] puis le mélange a été vortexé et centrifugé. Les quantités et les volumes indiqués ci-dessus correspondent à un puits et ont en fait été multipliés par trois et distribués dans trois puits. Après 10 min, le mélange a été ajouté sur les cellules et le surnageant a été réparti de façon homogène par une légère rotation manuelle horizontale. Tout de suite après, la plaque de culture a été centrifugée (Sigma 3K10, Bioblok, France), pendant 5 min à 1500 rpm (280 g). Après 2 à 3 heures d'incubation, 110 µl de sérum de veau foetal a été ajouté aux puits sans sérum. Les cellules ont été cultivées pendant 24 h, puis l'expression du gène rapporteur a été testée.

### - Mesure de la luciférase

L'expression du gène luciférase a été mesurée par luminescence. Le milieu de culture a été enlevé et le lysat cellulaire a été récolté après incubation, pendant 30 min, à température ambiante dans le tampon de lyse lx (Promega, USA). Le lysat a été vortexé doucement et centrifugé à 140 000 rpm (17 530 g), pendant 5 min, à 4°C. 20 µl de lysat ont été dilués dans 100 µl de tampon de réaction luciférase (Proméga, USA) et la luminescence a été mesurée pendant 10 sec (Mediators PhL, Vienne, Autriche). Les résultats ont été exprimés en unité de luminescence par mg de protéine (mesure par le test BCA, Pierce).

| **EXEMPLE 3 : Pourcentages de viabilité des cellules transfectées conformément à** **l'exemple 2.** | | | | | |
|---|---|---|---|---|---|
| **Dendrimères-N**^{**+**}**Et**_{**2**}**H, Cl**^{**-**} 5 équivalents 10 équivalents | | | **Dendrimères-N**^{**+**}**Et**_{**2**}**Me, CH**_{**3**}**CO**_{**2**}^{**-**} 5 équivalents 10 équivalents | | |
| G₁ | 95 | 105 | G₁ | 90 | 88 |
| G₂ | 91 | 83 | G₂ | 91 | 101 |
| G₃ | 83 | 90 | G₃ | 87 | 99 |
| G₄ | 94 | 91 | G₄ | 99 | 95 |
| G₅ | 90 | 80 | G₅ | 108 | 92 |
| PEI linéaire : 5 équivalents : 69 ; 10 équivalents : 84 | | | | | |
| PEI réticulé : 5 équivalents : 102 ; 10 équivalents : 91 | | | | | |

### RÉFÉRENCES

- [1]: A. D. Miller, *Nature* **1992**, *357*, 455-460.
- [2]: J. M. Wilson, *New Engl. J. Med.* **1996**, *334*, 1185-1187.
- [3]: P. Briand, A. Kahn, *Path. Biol.* **1993**, *41*, 663-671.
- [4]: B. Y. Roessler, J. W. Hartman, D. K. Vallance, Y. M. Latta, J. L. Janich, B. L. Davidson, *Hum. Gène. Ther.* **1995**, *6*, 307-316.
- [5]: C. J. Wheeler, J. H. Felgner, Y. J. Tsai, J. Marshal, L. Sukhu, S. G. Doh, J. Hartikka, J. Nietsupski, M. Manthorpe, M. Nichols, M. Plewe, X. Liang, J. Norman, A. Smith, S. H. Cheng, *Proc. Natl. Acad. Sci. USA* **1996**, *93*, 11454-11459.
- [6]: J. P. Behr, *Bioconjugate Chem.* **1994**, *5*, 382-389.
- [7]: J. P. Vigneron, N. Oudrhiri, M. Fauquet, L. Vergely, J. C. Bradley, M. Bassville, P. Lehn, J. M. Lehn, *Proc. Natl. Acad. Sci. USA* **1996**, *93*, 9682-9686.
- [8]: G. Byk, C. Dubertret, V. Escriou, M. Frederic, G. Jaslin, R. Rangara, B. Pitard, J. Crouzet, P. Wils, B. Schwartz, D. Scherman, *J. Med. Chem.* **1998**, *41*, 224-235.
- [9]: A. W. Miller, *Angew. Chem. Inter. Ed. Engl.* **1998**, *37*, 1768-1785.
- [10]: T. Hara, Y. Tan, L. Huang, *Proc. Natl. Acad. Sci. USA* **1997**, *94*, 14547-14552.
- [11]: I. Koltover, T. Salditt, J. O. Rädler, C. R. Safinya *Science* **1998**, *281,* 78-81.
- [12]: O. Boussif, M. A. Zanta, A. Adib, J. P. Behr, *Gène Ther.* **1996,** *3,* 1074-1080.
- [13]: J. Haensler, F. C. Szoka, *Bioconjugate Chem.* **1993,** *4,* 372-379.
- [14]: A. Bielinska, J. F. Kukowska-Latallo, J. Johnson, D. A. Tomalia, J. R. Baker, *Nucleic Acids Res.* **1996**, *24*, 2176-2182.
- [15]: M. X. Tang, C. T. Redemann, F. C. Szoka, *Bioconjugate Chem.* **1996**, *7*, 703-714.
- [16]: N. Launay, A. M. Caminade, R. Lahana, J. P. Majoral, *Angew. Chem. Int. Ed. Engl.* **1994**, *33*, 1589-1592.
- [17]: N. Launay, A. M. Caminade, J. P. Majoral, *J. Am. Chem. Soc.* **1995**, *117*, 3282-3283.
- [18]: C. Galliot, C. Larré, A. M. Caminade, J. P. Majoral, *Science* **1997**, *277*, 1981-1984.
- [19]: C. Larré, B. Donnadieu, A. M. Caminade, J. P. Majoral, *J. Am. Chem. Soc.* **1998**, *120*, 4029-4030.
- [20]: D. A. Tomalia, H. D Durst, *Topics Curr. Chem.* **1993**, *165,* 193-313.
- [21]: F. Zeng, S. C. Zimmerman, *Chem. Rev.* **1997**, *97*, 1681-1712.
- [22]: K. W. Pollak, J. W. Leon, J. M. J. Fréchet, M. Maskus, H. D. Abruna, *Chem. Mater.* **1998**, *10*, 30-38.
- [23]: E. Wagner, M. Cotten, R. Foisner, M. L. Birnstiel, *Proc. Natl. Acad. Sci. USA*, **1991**, *88,* 4255-4259.
- [24]: E. Wagner, C. Plank, K. Zatloukal, M. Cotten, M. L. Bimstiel, *Proc. Natl. Acad. Sci. USA* **1992**, *89*, 7934-7938.
- [25]: J. F. Kukowska-Latallo, A. U. Bielinska, J. Johnson, R. Spindler, D. A. Tomalia, J. R. Baker, *Proc. Natl. Acad. Sci. USA* **1996**, *93*, 4897-4902.
- [26]: J. S. Remy, A. Kichler, V. Mordinov, F. Schuber, J. P. Behr, *Proc. Natl. Acad. Sci. USA* **1995**, *92*, 1744-1748.
- [27]: Demande Internationale PCT WO 95/02397.
- [28]: D. Prévôté et al., *J. Org. Chem.,* **1997**, *62*, 14, 4834-4841.

## Revendications

1. Dendrimères phosphorés polycationiques, **caractérisés en ce qu'**ils sont constitués :
- d'une couche centrale sous la forme d'un noyau central P₀ comprenant 2 à 8 groupements fonctionnalisés,
- de n couches intermédiaires, identiques ou différentes, chacune desdites couches intermédiaires étant constituée d'unités P₁ répondant à la formule II : 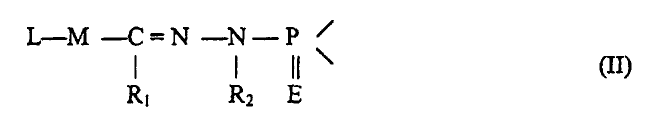
dans laquelle :
**L** est un atome d'oxygène, de phosphore ou de soufre,
**M** représente l'un des groupes suivants :
• un groupe aromatique di-, tri- ou tétrasubstitué par des groupes alkyles, des groupes alcoxy, des groupements insaturés du type oléfinique en C₁-C₁₂, azoïque, acétylènique, tous ces groupes pouvant incorporer ou non des atomes de phosphore, d'oxygène, d'azote, de soufre ou des halogènes, ou
• un groupe alkyle ou alcoxy comportant plusieurs substituant tels que définis lorsque M est un groupe aromatique,
**R**_{**1**} **et R**_{**2**}**,** qui peuvent être identiques ou différents, représentent un atome d'hydrogène ou l'un des groupes suivants : alkyle, alcoxy, aryle, comportant ou non des atomes de phosphore, d'oxygène, de soufre, d'azote ou des halogènes avec R₂ étant le plus souvent différent de R₁,
**n** est un nombre entier compris entre 1 et 11,
**E** est un atome d'oxygène, de soufre ou d'azote, ledit atome d'azote pouvant être lié à un groupe alkyle, alcoxy ou aryle, tous ces groupes pouvant incorporer ou non des atomes de phosphore, d'oxygène, d'azote, de soufre ou des halogènes,
- une couche externe constituée d'unités P₂, identiques ou différentes, et répondant à la formule III : 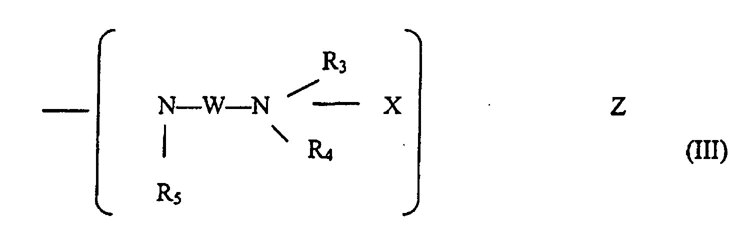
dans laquelle :
**R**_{**5**} représente un atome d'hydrogène ou l'un des groupes suivants : alkyle, alcoxy, aryle, ces groupes comportant ou non des atomes de phosphore, d'oxygène, d'azote, de soufre ou des halogènes,
**W** représente l'un des groupes suivants : alkyle, alcoxy, aryle, tous ces groupes comportant ou non des atomes de phosphore, d'oxygène, d'azote, de soufre ou des halogènes.
**R**_{**3**} **et R**_{**4**}**,** qui peuvent être identiques ou différents, représentent un groupe alkyle en C₁-C₅,
**X** représente un atome d'hydrogène ou un groupe alkyle en C₁-C₅ ou est absent, et
**Z** représente un ion halogènure, un groupe alkylCOO⁻ ou tout autre groupement anionique comportant des atomes de carbone, d'oxygène, de soufre, d'azote, de phosphore ou d'halogènes, ou est absent.

2. Dendrimères selon la revendication 1, **caractérisés en ce que** le noyau central P₀ est sélectionné dans le groupe constitué par le groupement de formule générale Ia : 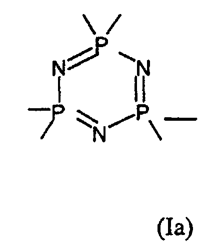 ou le groupement de formule générale Ib :

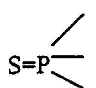

3. Dendrimères selon la revendication 1 ou la revendication 2, **caractérisés en ce qu'**ils présentent des unités terminales de formule III, dans lesquelles X représente un atome d'hydrogène, R₅ est un atome d'hydrogène, W représente un groupe (CH₂)₂, R₃ et R₄ sont identiques et représentent des groupes éthyle, Z est un ion chlorure et n est un nombre entier compris entre 1 et 11.

4. Dendrimères selon la revendication 1 ou la revendication 2, **caractérisés en ce qu'**ils présentent des unités terminales de formule III, dans lesquelles X représente un groupe méthyle, R₅ est un atome d'hydrogène, W représente un groupe (CH₂)₂, R₃ et R₄ sont identiques et représentent des groupes éthyle, Z est un groupe CH₃COO⁻ et n est un nombre entier compris entre 1 et 11.

5. Composition capable d'agir comme agent de transfection d'une séquence d'acide nucléique vers une cellule eucaryote, **caractérisée en ce qu'**elle comprend un acide nucléique et un dendrimère phosphoré polycationique selon l'une quelconque des revendications 1 à 4, couplé audit acide nucléique.

6. Composition selon la revendication 5, **caractérisée en ce qu'**elle comprend en outre au moins un véhicule pharmaceutiquement acceptable.

7. Composition selon la revendication 5 ou la revendication 6, **caractérisée en ce que** le rapport N/P, dans lequel N correspond aux groupes cationiques terminaux du dendrimère (amines chargées) et P correspond aux groupes phosphates dudit acide nucléique, est compris entre 5 et 10.

8. Composition selon l'une quelconque des revendications 5 à 7, **caractérisée en ce qu'**elle comprend en outre un agent de perméabilisation de la membrane capable de transporter ledit acide nucléique à travers les membranes cytoplasmiques ou endosomales de ladite cellule eucaryote.

9. Composition selon l'une quelconque des revendications 5 à 7, **caractérisée en ce que** ledit dendrimère phosphoré polycationique est associé de manière non-covalente avec ledit acide nucléique.

## Claims

1. Polycationic phosphorated dendrimers, **characterized in that** they consist of:
- a central layer in the form of a central ring P₀ comprising 2 to 8 functionalized groups,
- n intermediate layers, identical or different, each of said intermediate layers consisting of P₁ units having the formula II: 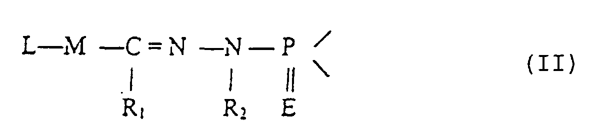
wherein:
**L** is an oxygen, phosphorus or sulphur atom,
**M** represents one of the following groups:
* an aromatic group di-, tri- or tetrasubstituted by alkyl groups, alkoxy groups, unsaturated groups of the C₁-C₁₂ olefinic, azoic, acetylenic type, all of which groups may or may not incorporate phosphorus, oxygen, nitrogen sulphur atoms or halides, or
* an alkyl or alkoxy group having a plurality of substituents as defined when M is an aromatic group,
**R**_{**1**} **and R**_{**2**}**,** which may be identical or different, represent a hydrogen atom or one of the following groups: alkyl, alkoxy, aryl, which may or may not include phosphorus, oxygen, sulphur, nitrogen atoms or halides with R₂ being most often different from R₁,
**n** is an integer between 1 and 11,
**E** is an oxygen, sulphur or nitrogen atom, said nitrogen atom being able to be bonded to an alkyl, alkoxy or aryl group, all of which groups may or may not incorporate phosphorus, oxygen, nitrogen, sulphur atoms or halides,
- an outer layer consisting of P₂ units, identical or different, and having the formula III: 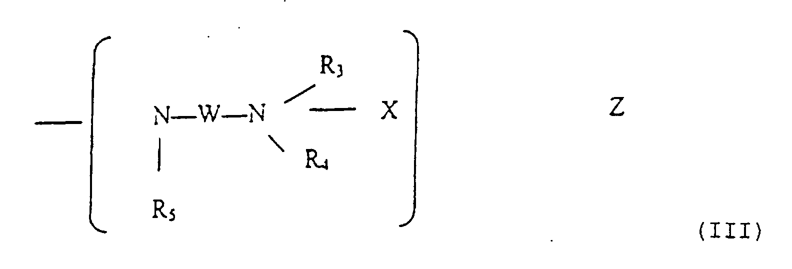
wherein:
**R**_{**5**} represents a hydrogen atom or one of the following groups H: alkyl, alkoxy, aryl, which groups may or may not include phosphorus, oxygen, nitrogen, sulphur atoms or halides,
**W** represents one of the following groups: alkyl, alkoxy, aryl, all of which groups may or may not include phosphorus, oxygen, nitrogen, sulphur atoms or halides.
**R**_{**3**} **and R**_{**4**}, which may be identical or different, represent a C₁-C₅ alkyl group,
**X** represents a hydrogen atom or a C₁-C₅ alkyl group or is absent, and
**Z** represents a halide ion, an alkylCOO⁻ group or any other anionic group having carbon, oxygen, sulphur, nitrogen, phosphorus atoms or halides, or is absent.

2. Dendrimers according to claim 1, **characterized in that** the central ring P₀ is selected from the group constituted by the group having the general formula Ia: 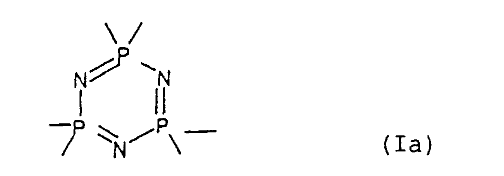 or the group having the general formula Ib :

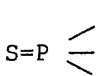

3. Dendrimers according to claim 1 or claim 2, **characterized in that** they have terminal units of formula III, wherein X represents a hydrogen atom, R₅ is a hydrogen atom, W represents a (CH₂)₂ group, R₃ and R₄ are identical and represent ethyl groups, Z is a chloride ion and n is an integer between 1 and 11.

4. Dendrimers according to claim 1 or claim 2, **characterized in that** they have terminal units of formula III, wherein X represents a methyl group, R₅ is a hydrogen atom, W represents a (CH₂)₂ group, R₃ and R₄ are identical and represent ethyl groups, Z is a CH₃COO⁻ group and n is an integer between 1 and 11.

5. A composition capable of acting as an agent for transfection of a nucleic acid sequence to an eukaryotic cell, **characterized in that** it comprises a nucleic acid and a polycationic phosphorated dendrimer according to any one of claims 1 to 4, coupled to said nucleic acid.

6. A composition according to claim 5, **characterized in that** it further comprises at least one pharmaceutically acceptable carrier.

7. A composition according to claim 5 or claim 6, **characterized in that** the N/P ratio, wherein N corresponds to the terminal cationic groups of the dendrimer (charged amines) and P corresponds to the phosphate groups of said nucleic acid, is between 5 and 10.

8. A composition according to any one of claims 5 to 7, **characterized in that** it further comprises a membrane permeabilising agent capable of transporting said nucleic acid through the cytoplasmic or endosomal membranes of said eukaryotic cell.

9. A composition according to any one of claims 5 to 7, **characterised in that** said polycationic phosphorated dendrimer is associated non-covalently with said nucleic acid.

## Patentansprüche

1. Phosphor enthaltende polykationische Dendrimere, **dadurch gekennzeichnet, daß** sie bestehen aus
- einer zentralen Schicht in Form eines zentralen Kerns P₀ umfassend 2 bis 8 funktionelle Gruppen;
- n identischen oder verschiedenen Zwischenschichten, wobei die Zwischenschichten bestehen aus Einheiten P₁, die der Formel II gehorchen 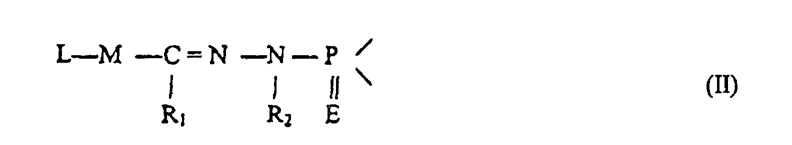
worin
L ein Sauerstoff-Atom, Phosphor-Atom oder Schwefel-Atom ist;
M für eine der folgenden Gruppen steht:
· eine durch Alkyl-Gruppen, Alkoxy-Gruppen, ungesättigte Gruppen des Typs C₁- bis C₁₂-Olefin, Azo-Gruppen, acetylenische Gruppen di-, tri- oder tetrasubstituierte aromatische Gruppe, wobei alle diese Gruppen Phosphor-Atome, Sauerstoff-Atome, Stickstoff-Atome, Schwefel-Atome oder Halogene einschließen können oder nicht, oder
· eine Alkyl-Gruppe oder Alkoxy-Gruppe, die mehrere Substituenten umfassen kann, wie sie definiert sind, wenn M eine aromatische Gruppe ist;
R₁ und R₂, die identisch oder verschieden sein können, für ein Wasserstoff-Atom oder eine der folgenden Gruppen stehen: Alkyl-Gruppe, Alkoxy-Gruppe, Aryl-Gruppe, umfassend Phosphor-Atome, Sauerstoff-Atome, Schwefel-Atome, Stickstoff-Atome oder Halogene, wobei R₂ am häufigsten verschieden von R₁ ist;
n eine ganze Zahl zwischen 1 und 11 ist;
E ein Sauerstoff-Atom, Schwefel-Atom oder Stickstoff-Atom ist, wobei das Stickstoff-Atom gebunden sein kann an eine Alkyl-Gruppe, Alkoxy-Gruppe oder Aryl-Gruppe, wobei alle diese Gruppen Phosphor-Atome, Sauerstoff-Atome, Stickstoff-Atome, Schwefel-Atome oder Halogene einschließen können oder nicht;
- eine Außenschicht, die aus identischen oder verschiedenen Einheiten P₂ besteht, die der Formel III gehorchen: 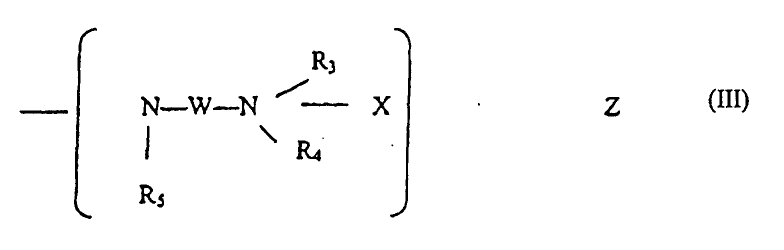
worin
R₅ für ein Wasserstoff-Atom oder eine der folgenden Gruppen steht: Alkyl-Gruppe, Alkoxy-Gruppe, Aryl-Gruppe, wobei diese Gruppen Phosphor-Atome, Sauerstoff-Atome, Stickstoff-Atome, Schwefel-Atome oder Halogene umfassen können oder nicht,
W für eine der folgenden Gruppen steht: Alkyl-Gruppe, Alkoxy-Gruppe, Aryl-Gruppe, wobei diese Gruppen Phosphor-Atome, Sauerstoff-Atome, Stickstoff-Atome, Schwefel-Atome oder Halogene umfassen können oder nicht,
R₃ und R₄, die identisch oder verschieden sein können, für eine C₁- bis C₅-Alkyl-Gruppe stehen,
X für ein Wasserstoff-Atom oder eine C₁- bis C₅-Alkyl-Gruppe steht oder fehlt, und
Z für ein Halogenid-Ion, eine Gruppe Alkyl-COO⁻ oder jede andere anionische Gruppe steht, die Kohlenstoff-Atome, Sauerstoff-Atome, Schwefel-Atome, Stickstoff-Atome, Phosphor-Atome oder Halogene umfaßt, oder fehlt.

2. Dendrimere nach Anspruch 1, **dadurch gekennzeichnet, daß** der zentrale Kern P₀ gewählt ist aus der Gruppe, die besteht aus der Gruppe der allgemeinen Formel Ia 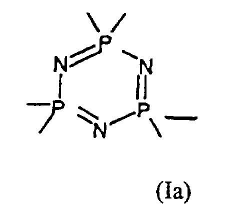 oder der Gruppe der allgemeinen Formel Ib:

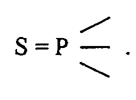

3. Dendrimere nach Anspruch 1 oder Anspruch 2, **dadurch gekennzeichnet, daß** sie terminale Einheiten der Formel III zeigen, in denen X für ein Wasserstoff-Atom steht, R₅ ein Wasserstoff-Atom ist, W für eine Gruppe (CH₂)₂ steht, R₃ und R₄ identisch sind und für Ethyl-Gruppen stehen, Z ein Chlorid-Ion ist und n eine ganze Zahl zwischen 1 und 11 ist.

4. Dendrimere nach Anspruch 1 oder Anspruch 2, **dadurch gekennzeichnet, daß** sie terminale Einheiten der Formel III zeigen, in denen X für eine Methyl-Gruppe steht, R₅ ein Wasserstoff-Atom ist, W für eine Gruppe (CH₂)₂ steht, R₃ und R₄ identisch sind und für Ethyl-Gruppen stehen, Z eine Gruppe CH₃COO⁻ ist und n eine ganze Zahl zwischen 1 und 11 ist.

5. Zubereitung, die als Mittel zur Transfektion einer Nukleinsäure-Sequenz auf eine Eukaryonten-Zelle wirken kann, **dadurch gekennzeichnet, daß** sie eine Nukleinsäure und ein Phosphor enthaltendes polykationisches Dendrimer nach einem der Ansprüche 1 bis 4 umfaßt, das an die Nukleinsäure gekoppelt ist.

6. Zubereitung nach Anspruch 5, **dadurch gekennzeichnet, daß** sie außerdem wenigstens ein pharmazeutisch annehmbares Vehikel umfaßt.

7. Zubereitung nach Anspruch 5 oder Anspruch 6, **dadurch gekennzeichnet, daß** das Verhältnis N / P worin N den kationischen Endgruppen des Dendrimers (geladene Amine) entspricht und P den Phosphat-Gruppen der Nukleinsäure entspricht, zwischen 5 und 10 liegt.

8. Zubereitung nach einem der Ansprüche 5 bis 7, **dadurch gekennzeichnet, daß** sie außerdem ein Mittel zum Durchlässigmachen der Membran umfaßt, das in der Lage ist, die Nukleinsäure durch die zytoplasmatischen oder endosomalen Membranen der Eukaryontenzelle zu transportieren.

9. Zubereitung nach einem der Ansprüche 5 bis 7, **dadurch gekennzeichnet, daß** das Phosphor enthaltende polykationische Dendrimer in nicht-kovalenter Weise mit der Nukleinsäure assoziiert ist.
